# EUROPEAN PATENT APPLICATION

(11) **EP 4 477 745 A1**
(43) Date of publication of application: **18.12.2024**
(21) Application number: 23753087.8
(22) Date of filing: 06.02.2023
(51) Int. Cl.: C12N 5/071

(54) **TWO-DIMENSIONAL CULTURE METHOD HAVING CLEAR CHEMICAL COMPOSITION FOR CULTURING THREE-DIMENSIONAL INTESTINAL ORGANOID-DERIVED INTESTINAL STEM CELL AGGREGATE**

(30) Priority: 09.02.2022 KR 20220016714
(71) Applicant: Korea Research Institute of Bioscience and Biotechnology, Daejeon 34141 (KR)
(72) Inventor: SON, Mi-Young, Daejeon 34141 (KR); KWON, Oh Man, Daejeon 34141 (KR); KIM, Dae Soo, Daejeon 34141 (KR); LEE, Hana, Daejeon 34141 (KR)
(74) Representative: Impact Intellectual Property LLP
(86) International application number: PCT/KR2023/001634
(87) International publication number: WO 2023/153737

(57) **Abstract**

The present disclosure relates to a method for two-dimensionally culturing an intestinal stem cell population in a chemically defined medium and a use thereof, and also relates to a method for differentiating the intestinal stem cell population into 2.5-dimensional intestinal epithelial cells and a use thereof.

## Description

### [Technical Field]

The present disclosure relates to a method for two-dimensionally culturing an intestinal stem cell population in a chemically defined medium, and a use thereof, and relates to a method for differentiating an intestinal stem cell population into 2.5-dimensional intestinal epithelial cells, and a use thereof.

### [Background Art]

Intestinal organoids are cell model systems that can model human intestinal development and disease, and have been spotlighted as excellent cell models that can be used in various fields, such as the study of physiological/pathological functions of intestine, the development of therapeutic agents for the treatment of intestinal diseases, and the evaluation of efficacy and toxicity of new drug candidate substances.

In addition, the intestinal organoids are in the spotlight as therapeutic agents that can regenerate a lesion by transplanting organoids into the intestine that is damaged or have not developed properly due to intestinal diseases. The intestinal organoids are composed of various cells including intestinal stem cells, which are key components of regeneration, and have been confirmed to efficiently regenerate damaged tissues by engrafting the intestinal organoids in the lesion, so that the possibility of development as a therapeutic agent continues to increase.

Recently, it has been repeatedly confirmed in several groups that human intestinal organoids transplanted into models with damaged intestinal tissue regenerate the damaged intestinal tissue, and the transplanted intestinal organoids have been demonstrated to be able to regenerate the structure and function of actual intestinal tissue at a high level.

However, in order for intestinal organoids to be utilized as a cell model system for various purposes or as a cell therapeutic agent for regeneration of damaged intestinal tissue, homogeneous intestinal organoids whose shape and function are adjustable within a certain range have to be necessarily mass-cultured, and a culture system that facilitates long-term culture and cryopreservation is required.

Currently, intestinal organoids are cultured through a three-dimensional culture using Matrigel, which is produced by isolating extracellular matrix produced from sarcoma transplanted to animals. However, such a culture has an intensified difference in the shape and function of the intestinal organoid between batches, resulting in disadvantages of low homogeneity, difficulty in large-scale culture, impossibility of long-term culture and cryopreservation, and high culture cost.

Accordingly, it is necessary to develop a novel method for culturing intestinal organoids, which enables long-term culture and cryopreservation of homogeneous intestinal organoids and can develop the intestinal organoids as a cell model system or a cell therapeutic agent with little difference between batches because of stable cell supply through large-scale culture.

Under the background described above, the present inventors have developed a method for culturing cells, which can easily and rapidly culture the cells by isolating only intestinal stem cells from human pluripotent stem cell-derived three-dimensional intestinal organoids and then culturing the intestinal stem cells in a chemically defined culture medium, and enables large-scale culture, cryopreservation, and thawing through long-term stable subculture. Therefore, it is confirmed that homogeneous intestinal stem cells and various uses may be provided, thereby completing the present disclosure.

### [Disclosure]

### [Technical Problem]

An object of the present disclosure is to provide a method for culturing an intestinal stem cell population, which two-dimensionally cultures a single cell or a small cell clump isolated from pluripotent stem cell-derived three-dimensional intestinal organoids in a chemically defined culture medium.

Another object of the present disclosure is to provide a method for producing intestinal epithelial cells, which cultures the intestinal stem cell population in a chemically defined differentiation medium through air-liquid interface culture.

Still another object of the present disclosure is to provide a use of the above-described intestinal stem cell population and/or intestinal epithelial cells.

### [Technical Solution]

Hereinafter, description of the overlapping contents will be omitted to prevent any potential confusion arising from redundancy. In other words, the contents of the invention are not limited to the following contents, and should be interpreted according to the overall contents of the invention.

The terms used herein are only for the purpose of describing particular embodiments and are not intended to limit the present disclosure. The singular expression also includes the plural meaning as long as it does not differently mean in the context. In addition, the terms "comprise", "have" and the like, herein are used to indicate that there are features, numbers, steps, elements, or combination thereof, and they should not exclude the possibilities of combination or addition of one or more features, numbers, operations, elements, or a combination thereof.

It should be understood that when an amount, concentration, or other value or parameter as used herein is given as an enumeration of a range, a preferable range, or preferable upper and lower values, all ranges formed with any upper limit or preferable values of any one pair and any lower limit or preferable values of any one pair are specifically disclosed, regardless of whether the range is disclosed separately.

When a range of numerical values is described herein, unless otherwise stated, it is intended that endpoints of a range and the scope of the present disclosure within the range are not limited to the specific values described when defining the range.

### Pluripotent Stem Cells

The term "stem cell" as used herein refers to a cell that has the ability to continue proliferation into undifferentiated cells, that is, the ability to replicate itself, and has the ability to differentiate into various types of specific cells constituting a tissue or organ from all three germ layers.

As used herein, the term "pluripotent stem cell (PSC)" is commonly known as a PS cell, and includes any cell that can be differentiated into almost all cells, i.e., a cell derived from any of three germ layers (reproductive epithelium) including endoderm (internal gastric wall, gastrointestinal tract, lung), mesoderm (muscle, bone, blood, genitourinary), and ectoderm (epithelial tissue and nervous system). The PSC may be derived from embryonic stem cells (including embryonic germ cells) or may be a descendant of pluripotent cells obtained by forcing expression of specific genes in non-pluripotent cells such as adult somatic cells or fully differentiated cells.

As used herein, the term "induced pluripotent stem cell (iPSC)" is commonly abbreviated as an iPS cell, and refers to a type of pluripotent stem cells that are normally and artificially induced from non-pluripotent cells, such as adult somatic cells, by inducing "forced" expression of specific genes.

As used herein, the term "embryonic stem cell (ESC)" is commonly abbreviated as an ES cell, and refers to a cell derived from an inner cell mass of blastocyst, which is a pluripotent early-stage embryo. For purposes of the present disclosure, the term "ESC" is sometimes used broadly, including embryonic germ cells.

### Basal Medium

The medium used in the method of the present disclosure is any basal medium suitable for animal or human cells, to which limitations provided herein are applied.

The basal medium for animal or human cell culture typically contains a number of components necessary to support maintenance of cultured cells. Combinations of suitable components may be easily formulated by a skilled person in consideration of the following contents. The basal medium for use in the present disclosure will generally include a nutrient solution containing standard cell culture components as described in more detail above and in the document, for example, amino acids, vitamins, lipid supplements, mineral salts, carbon energy sources, and buffers. In some embodiments, the culture medium is further supplemented with one or more standard cell culture components selected from, for example, amino acids, vitamins, liquid supplements, inorganic salts, carbon energy sources, and buffers.

The skilled person will understand, from common general knowledge, the type of culture medium that may be used as the basal medium in the differentiation medium of the present disclosure. The potentially suitable cell culture medium include commercially available, but is not limited to, Dulbecco's modified eagle medium (DMEM), minimum essential medium (MEM), knockout-DMEM (KO-DMEM), Glasgow's minimum essential medium (G-MEM), a basal medium eagle (BME), DMEM/Ham's F12, Advanced DMEM/Ham's F12, Iscove's modified dulbecco's medium, minimum essential medium (MEM), Ham's F-10, Ham's F-12, Medium 199, RPMI 1640 medium, and KnockOut Serum replacement XenoFree medium

More specifically, the cell culture medium may be selected from the group consisting of RPMI 1640, Dulbecco's modified eagle medium (DMEM), Ham's F12, DMEM/F12, DMEM/F12 and Advanced DMEM/F12.

For example, the basal medium may be DMEM/F12, Advanced DMEM/F12 and/or RPMI 1640. As needed, Advanced DMEM/F12 or Advanced RPMI that is optimized for serum-free culture and already contains insulin is used.

### Method for Culturing Intestinal Stem Cell Population

The present disclosure provides a method for culturing an intestinal stem cell population, which two-dimensionally cultures a single cell or a small cell clump isolated from pluripotent stem cell-derived three-dimensional intestinal organoids in a chemically defined culture medium.

Specifically, there is provided a method for culturing an intestinal stem cell population, the method comprising: (a) isolating a pluripotent stem cell-derived three-dimensional intestinal organoid into a single cell or a small cell clump; and
(b) two-dimensionally culturing the single cell or the small cell clump in a culture medium comprising a WNT/R-spondin activator, an activator of a prostaglandin signaling pathway, and a receptor tyrosine kinase ligand.

As used herein, the term "organoid" refers to a three-dimensional population of one or more cell types that mimics a physical appearance, an actual structure, or a function of a tissue or an organ.

The three-dimensional intestinal organoid of the present disclosure is derived from pluripotent stem cells, in which the three-dimensional intestinal organoid may be produced through the steps of:
(a) culturing pluripotent stem cells in a medium comprising any one or more selected from the group consisting of Nodal, Activin A, Activin B, BMP4, CHIR99021, Wnt3a, and bFGF to differentiate the stem cells to definitive endoderm;
(b) culturing the definitive endoderm in a medium comprising: any one or more GSK3 inhibitors selected from the group consisting of BIO(6-bromoindirubin-3'-oxime), SB216763 (3-(2,4-dichlorophenyl)-4-(1-methyl-1H-indol-3-yl)-1H-pyrrole-2,5-dione), GSK-3β inhibitor VII(α,4-dibromoacetophenone), L803-mts(Myr-N-GKEAPPQSpP-NH₂), and CHIR99021; and a fibroblast growth factor (FGF), to differentiate the definitive endoderm into three-dimensional hindgut spheroids; and
(c) culturing the three-dimensional hindgut spheroids in a medium comprising: a BMP inhibitor; a WNT/R-spondin activator; a receptor tyrosine kinase ligand; and any one or more factors selected from the group consisting of IL-2, IL-22, IL-6, IL-1β, IL-11, EGF, OSM, NRG-1, IL-10, and colivelin, to produce a three-dimensional intestinal organoid.

The differentiation of intestinal organoids from pluripotent stem cells goes through: 1) a differentiation process of pluripotent stem cells into definitive endoderm (DE) cells, 2) a differentiation process of definitive endoderm (DE) cells to hindgut (HG), and 3) a differentiation process of hindgut (HG) into (mature) intestinal organoids.

### 1) Step of Differentiating Pluripotent Stem Cells into Definitive Endoderm (DE) Cells

One or more growth factors are used in a differentiation process from pluripotent stem cells to definitive endoderm(DE) cells.

Specifically, growth factors that may affect Activin A/nodal signaling activation or phosphatidylinositol 3-kinase (PI3K) signaling inhibition may be used.

For example, one or more growth factors used in the differentiation process may comprise a growth factor in TGF-β superfamily. One or more growth factors may comprise Nodal/Activin and/or BMP subgroup in growth factors of TGF-beta super family.

In some aspects, one or more growth factors are selected from the group consisting of Nodal, Activin A, Activin B, BMP4, CHIR99021, Wnt3a, bFGF or any combination of these growth factors.

In some aspects, the pluripotent stem cells are treated with one or more growth factors for 6 hours or longer; 12 hours or longer; 18 hours or longer; 24 hours or longer; 36 hours or longer; 48 hours or longer; 60 hours or longer; 72 hours or longer; 84 hours or longer; 96 hours or longer; 120 hours or longer; 150 hours or longer; 180 hours or longer; or 240 hours or longer. In some aspects, the embryonic stem cells or iPSCs are treated with the one or more growth factors at a concentration of 10 ng/ml or more; 20 ng/ml or more; 50 ng/ml or more; 75 ng/ml or more; 100 ng/ml or more; 120 ng/ml or more; 150 ng/ml or more; 200 ng/ml or more; 500 ng/ml or more; 1,000 ng/ml or more; 1,200 ng/ml or more; 1,500 ng/ml or more; 2,000 ng/ml or more; 5,000 ng/ml or more; 7,000 ng/ml or more; 10,000 ng/ml or more; or 15,000 ng/ml or more. In some aspects, the concentration of the growth factor is maintained at a constant level during the treatment process. The concentration of the growth factor may be varied during the treatment process.

That is, the method may comprise a step of treating pluripotent stem cells with any one or more selected from the group consisting of Nodal, Activin A, Activin B, BMP4, CHIR99021, bFGF, and Wnt3a to differentiate the pluripotent stem cells to definitive endoderm cells. More specifically, the method may comprise a step of treating embryonic stem cells or induced pluripotent stem cells with any one selected from the group consisting of Activin A, BMP4, CHIR99021, bFGF, and Wnt3a to differentiate the embryonic stem cells or induced pluripotent stem cells to definitive endoderm cells. More specifically, the method may comprise a step of treating embryonic stem cells or induced pluripotent stem cells with Activin A to differentiate the embryonic stem cells or induced pluripotent stem cells to definitive endoderm cells.

Although the present disclosure is not limited thereto, the factors may be used, for example, in an amount of 1 ng/ml to 1,000 ng/ml, preferably 10 ng/ml to 500 ng/ml.

According to one embodiment of the present disclosure, Activin A may be used to differentiate pluripotent stem cells to definitive endoderm (DE) cells. In the differentiation, for example, 10 ng/ml to 500 ng/ml of Activin A may be used, but the present disclosure is not limited thereto. More specifically, 10, 20, 30, 40, 50, 60, 70, 80, 90, 100, 200, 300, 400, or 500 ng/ml of Activin A may be used. More specifically, the factor may be cultured for 6 hours to 120 hours, or 12 hours to 100 hours, but the present disclosure is not limited thereto.

According to one embodiment of the present disclosure, the growth factor is suspended in a medium comprising fetal bovine serum (FBS) with varying concentrations, as needed. For example, the medium may comprise any fetal bovine serum (FBS or FCS) suitable for growth, such as 0.1, 0.2, 0.3, 0.5, 1.0, 2.0, 3.0, 4.0, or 5.0%.

As needed, the cells may be cultured by contacting extracellular matrix. The extracellular matrix includes all of the matters described herein and may be applied to the present differentiation method.

### 2) Step of Differentiating Definitive Endoderm (DE) Cells into Three-Dimensional Hindgut (HG) Spheroids

The definitive endoderm (DE), which has undergone a process of producing definitive endoderm induced by the above-described factors, preferably induced by Activin, more preferably, induced by Activin A, may undergo a step of differentiation into Hindgut (HG) by an FGF/Wnt signal.

FGF and Wnt ligands may differentiate DE developed from iPSC or ESC directly into Hindgut.

The differentiation into Hindgut or Mid-hindgut may be performed through three-dimensional culture, spheroid culture, or the like, as needed. For example, the differentiation into Hindgut or Mid-hindgut may be performed on a micropatterned substrate/plate (e.g., Aggrewell, EZSPHERE) and formed into aggregation (e.g., spheroid) form or may be performed through three-dimensional culture (e.g., suspension) within a bioreactor. As for serum-free differentiation, B-27 may be added instead of fetal bovine serum (FBS or FCS). In addition, the culture medium may further comprise any one or more selected from the group consisting of Activin A, FGF, bFGF, BMP-4, LY294002 (PI3K inhibitor), CHIR99021 (GSK3 inhibitor), and Retinoic acid, but is not limited thereto.

Altering the expression of any Wnt signaling protein in combination with any FGF ligand may result in direct differentiation as described herein.

Modulators/activators of the Wnt signaling pathway include Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16. In some aspects, the pathway may be modulated through the use of a small molecule modulator or a protein modulator that activates the above-described pathway or a protein that activates the pathway. Examples of the small molecule modulator of the Wnt pathway include, but are not limited to, lithium chloride; 2-amino-4,6-disubstituted pyrimidine(hetero)arylpyrimidine; IQ1; QS11; NSC668036; DCA-beta catenin; and 2-amino-4-[3,4-(methylenedioxy)-benzylamino]-6-(3-methoxyphenyl) pyrimidine. Exemplary natural inhibitors of Wnt signaling include, but are not limited to, Dkk1, SFRP protein, and FrzB. In some aspects, exogenous molecules include, but are not limited to, small molecules such as WAY-316606; SB-216763; or BIO(6-bromoindirubin-3'-oxime).

In addition, the exogenous molecules include GSK3-inhibiting molecules or proteins that activate the Wnt signaling pathway. Exemplary GSK3 inhibitors include, but are not limited to, LY2090314, BIO(6-bromoindirubin-3'-oxime), SB216763 (3-(2,4-dichlorophenyl)-4-(1-methyl-1H-indol-3-yl)-1H-pyrrole-2,5-dione), GSK-3β inhibitor VII (α, 4-dibromoacetophenone), L803-mts (Myr-N-GKEAPPAPPQSpP-NH₂), and CHIR99021.

Specifically, the GSK3 inhibitors include Chiron/CHIR99021 that inhibits GSK3. The GSK3 inhibitor may be treated in an amount of about 0.1 uM to about 100 uM, or about 0.2 uM to about 50 uM, or about 0.3 uM to about 10 uM.

A Fibroblast growth factor (FGF) is a group of growth factors associated with angiogenesis, wound healing, and embryo development. In some aspects, it will be understood by one of ordinary skill in the art that any FGF may be used together with a protein derived from the Wnt signal pathway.

In some embodiments, the FGF signaling pathway is activated by contacting cells with at least one molecule selected from the group consisting of FGF1, FGF2, FGF3, FGF4, FGF10, FGF11, FGF12, FGF13, FGF14, FGF15, FGF16, FGF17, FGF18, FGF19, FGF20, FGF21, FGF22, and FGF23.

That is, it is possible to undergo the step of culturing the definitive endoderm in a medium comprising: any one or more GSK3 inhibitors selected from the group consisting of BIO(6-bromoindirubin-3'-oxime), SB216763 (3-(2,4-dichlorophenyl)-4-(1-methyl-1H-indol-3-yl)-1H-pyrrole-2,5-dione), GSK-3β inhibitor VII(a,4-dibromoacetophenone), L803-mts(Myr-N-GKEAPPQSp-NH₂), and CHIR99021; and a fibroblast growth factor (FGF), to differentiate the definitive endoderm into three-dimensional hindgut spheroids.

In some aspects, the DE cells are treated with one or more modulators of the signaling pathways described herein for 6 hours or longer; 12 hours or longer; 18 hours or longer; 24 hours or longer; 36 hours or longer; 48 hours or longer; 60 hours or longer; 72 hours or longer; 84 hours or longer; 96 hours or longer; 120 hours or longer; 150 hours or longer; 180 hours or longer; 200 hours or longer; 240 hours or longer; 270 hours or longer; 300 hours or longer; 350 hours or longer; 400 hours or longer; 500 hours or longer; 600 hours or longer; 700 hours or longer; 800 hours or longer; 900 hours or longer; 1,000 hours or longer; 1,200 hours or longer; or 1,500 hours or longer.

In some aspects, the DE cells are treated with one or more molecules of the FGF signaling pathway described herein at a concentration of 10 ng/ml or more; 20 ng/ml or more; 50 ng/ml or more; 75 ng/ml or more; 100 ng/ml or more; 120 ng/ml or more; 150 ng/ml or more; 200 ng/ml or more; 500 ng/ml or more; 1,000 ng/ml or more; 1,200 ng/ml or more; 1,500 ng/ml or more; 2,000 ng/ml or more; 5,000 ng/ml or more; 7,000 ng/ml or more; 10,000 ng/ml or more; or 15,000 ng/ml or more.

In some aspects, the concentration of signaling molecules remains constant during the course of treatment. In another aspect, the concentration of molecules in the signaling pathway is varied during the course of the treatment.

In some aspects, the signaling molecules according to the invention are suspended in a DMEM medium.

In some aspects, the signaling molecules according to the invention are suspended in a medium comprising fetal bovine serum (FBS or FCS).

Any known molecules of the signal pathway described herein, including any molecules of the Wnt and FGF signaling pathways, are applicable alone or in combination.

That is, the method comprises a step of treating FGF and Wnt ligands to differentiate the definitive endoderm cells to hindgut. More specifically, the method comprises a step of treating FGF and CHIR99021 to differentiate the definitive endoderm cells to the hindgut.

According to one embodiment of the present disclosure, the differentiation of the definitive endoderm (DE) to the hindgut may be performed through the GSK3 inhibitor and the fibroblast growth factor. More specifically, the differentiation may proceed to the hindgut through three-dimensional culture by treating CHIR99021 and FGF4.

In the differentiation, for example, 100 ng/ml, 120 ng/ml, 150 ng/ml, 200 ng/ml, 500 ng/ml, 1,000 ng/ml; or 1,200 ng/ml of FGF4 may be used, but the present disclosure is not limited thereto. Specifically, FGF4 may be used at a concentration of 100 ng/ml to 1,200 ng/ml, more specifically, 120 to 1,000 ng/ml.

In addition, CHIR99021 may be used in an amount of about 0.3 uM to about 10 uM. As needed, the factor is suspended in a medium comprising fetal bovine serum (FBS). For example, the medium may comprise any fetal bovine serum (FBS or FCS) suitable for growth, such as 0.1, 0.2, 0.3, 0.5, 1.0, 2.0, 3.0, 4.0, or 5.0%.

More specifically, the medium may be cultured for 24 hours or longer; 36 hours or longer; 48 hours or longer; 60 hours or longer; 72 hours or longer; 84 hours or longer; 96 hours or longer; 120 hours or longer; 150 hours or longer; 180 hours or longer, but the present disclosure is not limited thereto. Preferably, the medium may be cultured for 24 hours to 360 hours, and more preferably for 36 hours to 240 hours.

As needed, a BMP activator may be added and cultured to control a BMP signal. The BMP activator may be one of BMP2, BMP4, or both of BMP2 and BMP4, small molecules that activate a BMP pathway, and/or proteins that activate the BMP pathway.

As needed, the cells may be cultured by contacting extracellular matrix. The extracellular matrix includes all of the matters described herein and may be applied to the present differentiation method.

### 3) Step of Differentiating Three-Dimensional Hindgut (HG) Spheroids into Three-Dimensional Intestinal Organoids

The three-dimensional intestinal organoid of the present disclosure is a matured intestinal organoid, and refers to an intestinal organoid in which genes necessary for digestive functions, transport systems, immune functions, and host defense of the small intestine of an adult are expressed. Specifically, the small intestine of an adult has unique characteristics including an enhancement of expression of small intestine stem cell marker gene and genes necessary for digestive functions, transport systems, immune functions, and host defense. In particular, proper expression and activity of transporters involved in physiological and pharmacokinetic roles are a prerequisite for normal small intestinal functions, such as absorption, distribution and excretion of drugs.

Particularly, the three-dimensional intestinal organoid according to the present disclosure has improved efficacy in terms of engraftment. More specifically, this may be due to high expression characteristics of vascular endothelium-related factors in a mesenchymal stromal cell layer present around an intestinal epithelial cell layer constituting the organoid and various secreted factors that are characteristics of mesenchymal stromal cells.

In addition, the organoid may exhibit characteristics of budding structure development present on the intestinal organoid. In addition, the organoid exhibits higher development of goblet cells, thereby exhibiting high efficiency in terms of differentiation ability of intestinal stem cells.

In the differentiation of three-dimensional hindgut (HG) spheroids into three-dimensional intestinal organoids, BMP inhibitors, WNT/R-spondin activators, and receptor tyrosine kinase ligands are essentially included.

In addition, one or more additional components selected from the group consisting of a ROCK inhibitor, B27, N-Acetyl-L-cysteine (NAC), N2, nicotinamide, Gastrin, IGF-1, heregulin-1β, bFGF, A-83-01, and/or SB202190 may be included.

The BMP inhibitor is an agent that binds to BMP molecules to form a complex. The inhibitor may be an agent that binds to the BMP receptor and prevents binding of the BMP ligand to the receptor, such as an antibody that binds to the receptor. The BMP inhibitor may be a protein or a small molecule and may be natural, modified, and/or partially or entirely synthetic. The BMP inhibitor may be Noggin, DAN, or a DAN-like protein including Ceberus and Gremlin (R&D systems). A preferred BMP inhibitor is Noggin. Noggin may be used at any suitable concentration. The culture medium may include about 10 ng/ml to about 100 ng/ml of Noggin. For example, the culture medium may include about 10 ng/ml, 20 ng/ml 30 ng/ml 40 ng/ml, or 50 ng/ml or more of Noggin. Preferably, the culture medium may include approximately 10 to 100 ng/ml of Noggin.

Preferably, the WNT/R-spondin activator may be R-spondin 1, R-spondin 2, R-spondin 3, or R-spondin 4. The culture medium may include the WNT/R-spondin activator at a concentration of 50 ng/ml, 100 ng/ml, 200 ng/ml, 300 ng/ml, 500 ng/ml, 600 ng/ml, 700 ng/ml, 800 ng/ml, 900 ng/ml, 1 ug/ml, 1.5 ug/ml, or 2 ug/ml or more. Preferably, the culture medium may include approximately 50 to 800 ng/ml of R-spondin 1.

The receptor tyrosine kinase ligand is, for example, a mitogenic growth factor selected from the group consisting of an epidermal growth factor (EGF), a transforming growth factor-alpha (TGF-alpha), a basic fibroblast growth factor (bFGF), a brain-derived neurotrophic factor (BDNF), a hepatocyte growth factor (HGF), and a keratinocyte growth factor (KGF).

Preferably, the receptor tyrosine kinase ligand is the EGF. The EGF is a potent mitogen for a variety of cultured ectodermal and mesodermal cells and has sufficient effects on differentiation of certain cells in vivo and in vitro and some fibroblasts in cell cultures. Preferred concentrations thereof are at least 10, 20, 25, 30, 40, 45, or 50 ng/ml, and 100 ng/ml, 200 ng/ml, 300 ng/ml, 500 ng/ml, 600 ng/ml, and 700 ng/ml or more. More preferred concentrations are more than or equal to 50 ng/ml and less than or equal to 300 ng/ml.

In addition to the essential composition of the BMP inhibitor, the WNT/R-spondin activator, and the receptor tyrosine kinase ligand as described above, additional factors may be interpreted as further comprising addition of any known factor of the intestinal organoid.

Preferably, depending on the desired form desired of differentiation, one or more additional components selected from the group consisting of ROCK inhibitors, B27, N-acetyl-L-cysteine (NAC), N2, nicotinamide, Gastrin, IGF-1, heregulin-1β, bFGF, A-83-01, and SB202190 may be further included.

The ROCK inhibitor is preferably selected from R-(+)-trans-4-(1-aminoethyl)-N-(4-pyridyl)cyclohexanecarboxamide dihydrochloride monohydrate (Y-27632), 5-(1,4-diazepan-1-ylsulfonyl)isoquinoline (Fasudil or HA1077), and (S)-(+)-2-methyl 1-[(4-methyl-5-isoquinolinyl)sulfonyl]-hexahydro-1H-1,4-diazepinedihydrochloride (H-1 152).

N-acetyl-L-cysteine (NAC), N2, nicotinamide, Gastrin, IGF-1, heregulin-1β, bFGF, A-83-01, and/or SB202190 may be added as needed, for example, to improve the culture efficiency and lifespan of organoids, to modulate the proliferation of cells, to aid in the stability of DNA and the like.

Preferably, B27 may be further included. More specifically, the B27 supplement is referred to as "B27 supplement minus vitamin A" (also referred to herein as "B27 without vitamin A" or "B27 wo VitA"; and it may be found in Invitrogen (Located in Carlsbad, California, USA); www.invitrogen.com; current catalog number 12587010; and PAA Laboratories GmbH (Located in Pasching, Austria); www.paa.com; catalog number F01-002; and document [Brewer et al. (1993) J Neurosci Res.35(5):567-76]).

In some embodiments, the B27 supplement may be replaced with a generic formulation including one or more of the components selected from the following list: biotin, cholesterol, linoleic acid, linolenic acid, progesterone, putrescine, retinyl acetate, sodium selenite, tri-iodothyronine (T3), DL-alphatocopherol (vitamin E), albumin, insulin, and transferrin.

As needed, the cells may be cultured by contacting extracellular matrix upon differentiation of the three-dimensional hindgut (HG) spheroids into the three-dimensional intestinal organoids.

In some embodiments, the extracellular matrix is a three-dimensional matrix. In some embodiments, the hindgut is embedded in the extracellular matrix. The culture medium of the present disclosure may be diffused into the three-dimensional extracellular matrix.

The extracellular matrix disclosed in Polymer Hydrogels to Guide Organotypic and Organoid Cultures (Adv Funct Mater, 2020, Valentina Magno et al.) and Engineering the Extracellular Matrix for Organoid Culture (Int J Stem Cells. 2022 Feb 28; 15(1): 60-69) is incorporated herein by reference.

The extracellular matrix includes fibrin, laminin, collagen, and/or alginate, but is not limited thereto. Examples of extracellular matrix-producing cells include chondrocytes, which mainly produce collagen and proteoglycans; fibroblasts, which mainly produce type IV collagen, laminin, interstitial procollagen, and fibronectin; and colon myofibroblasts, which mainly produce collagen (types I, III, and V), chondroitin sulfate proteoglycans, hyaluronic acid, fibronectin, and tenascin-C. These are "naturally produced extracellular matrices". Naturally produced extracellular matrix may be commercially provided. Examples of commercially available extracellular matrices include extracellular matrix proteins (Invitrogen) and basement membrane preparations (e.g., Cultrex (registered trademark) basal membrane extracts (Trevigen, Inc.), type I collagen (Invitrogen), Vitrogel (registered trademark) (TheWell Bioscience Inc.), Geltrex (ThermoFisher), or Matrigel (trademark) (BD Biosciences)) from Engelbreth-Holm-Swarm (EHS) murine sarcoma cells.

The differentiation of three-dimensional hindgut (HG) spheroids into three-dimensional intestinal organoids essentially involves, in addition to the above-described factors of differentiation into the organoids, treatment with cytokines used in co-culture to mimic the in vivo intestinal enviroment or secreted in T-lymphocyte co-culture(that is, treatment with cytokines secreted by T-lymphocyte), and/or treatment with STAT3 and mTOR signaling pathway activators.

More specifically, the cytokine secreted by T-lymphocytes may be one or more selected from the group consisting of IL-2 (interleukin-2), IL-22 (interleukin-22), IL-6 (interleukin-6), IL-1β (interleukin-1β), IL-11 (interleukin-11), EGF (epidermal growth factor), OSM (oncostatin M), and IL-10 (interleukin-10), and more specifically, may be IL-2.

In addition to the above-described factors of differentiation into the organoids, STAT3 and mTOR signaling pathway activators may be treated to perform maturation of intestinal organoids. For example, the maturation of intestinal organoids may be performed by treatment with colivelin.

In addition, the maturation of intestinal organoids may be performed by treatment with NRG-1 (Neuregulin-1).

That is, the three-dimensional intestinal organoid may be produced through the step of: culturing the three-dimensional hindgut spheroid in a medium comprising a BMP inhibitor; a WNT/R-spondin activator; a receptor tyrosine kinase ligand; and any one or more factors selected from the group consisting of IL-2, IL-22, IL-6, IL-1β, IL-11, EGF, OSM, NRG-1, IL-10, and colivelin, to produce a three-dimensional intestinal organoid.

That is, at least one cytokine selected from the group consisting of IL-2, IL-22, IL-6, IL-1β, IL-11, EGF, OSM, and IL-10 for intestinal maturation directly in the hindgut, and/or colivelin treats the medium components, thereby greatly enhancing the degree of maturation of the organoids.

In other words, any one or more cytokines selected from the group consisting of IL-2, IL-22, IL-6, IL-1β, IL-11, EGF, OSM, and IL-10, and/or colivelin may be included as a medium component. Preferred concentrations thereof are at least 0.1, 0.2, 0.3, 0.4, 0.5, 0.6, 0.7, 0.8, 0.9, 1.0, 2.0, 3.0 ng/ml. More preferably, the concentration may be 0.3 to 2.0 ng/ml.

### Step of Isolating Pluripotent Stem Cell-Derived Three-Dimensional Intestinal Organoid into Single Cells or Small Cell Clumps

In the present disclosure, in the step of isolating the pluripotent stem cell-derived three-dimensional intestinal organoid into single cells or small cell clumps, the intestinal organoid may be dissociated by treating EDTA, trypsin, or both of EDTA and trypsin.

More specifically, it is preferable to treat EDTA, trypsin or both of EDTA and trypsin in a state in which the extracellular matrix remaining in the three-dimensional intestinal organoid is removed as much as possible to purify the isolated single cells or small cell clumps.

In this process, the three-dimensional intestinal organoid may be carefully isolated through pipetting or the like as needed, and more preferably, may be isolated close to the single cells.

The isolated single cells or the small cell clumps may be briefly cultured or washed in a basal medium, as needed, and may be cultured in the intestinal stem cell culture medium according to the present disclosure.

### Step of Performing Two-Dimensional Culture in Culture Medium Comprising WNT/R-Spondin Activator, Activator of Prostaglandin Signaling Pathway, and Receptor Tyrosine Kinase Ligand

A culture medium for producing an intestinal stem cell population used herein is provided to two-dimensionally culturing an intestinal stem cell population, and essentially comprises a WNT/R-spondin activator, an activator of a prostaglandin signaling pathway, and a receptor tyrosine kinase ligand.

The WNT/R-spondin activator serves to modulate stemness and proliferation of two-dimensional intestinal stem cells through activation of a WNT signaling system, and may be any one or more selected from the group consisting of R-spondin 1, R-spondin 2, R-spondin 3, R-spondin 4, and R-spondin mimetic substances. Preferably, WNT/R-spondin activator may be R-spondin 1.

The culture medium may include the WNT/R-spondin activator at a concentration of 50 ng/ml, 100 ng/ml, 200 ng/ml, 300 ng/ml, 500 ng/ml, 600 ng/ml, 700 ng/ml, 800 ng/ml, 900 ng/ml, 1 ug/ml, 1.5 ug/ml, or 2 ug/ml or more. Preferably, the culture medium may include approximately 50 to 800 ng/ml of R-spondin 1.

The activator of the prostaglandin signaling pathway serves to modulate proliferation of the intestinal stem cell population through activation of the prostaglandin signaling system, and may be any one or more selected from the group consisting of arachidonic acid (AA), prostaglandin E2 (PGE2), prostaglandin G2 (PGG2), prostaglandin F2 (PGF2), prostaglandin H2 (PGH2), and prostaglandin D2 (PGD2). Preferably, the activator of the prostaglandin signaling pathway may be prostaglandin E2 (PGE2).

As the activator of the prostaglandin signaling pathway, it is preferable that prostaglandin E2 may be treated in an amount of about 0.1 uM to about 100 uM, or about 0.2 uM to about 50 uM, or about 0.3 uM to about 10 uM.

The receptor tyrosine kinase ligand serves to prevent apoptosis of the intestinal stem cell population and modulate proliferation, and may be any one selected from the group consisting of an epidermal growth factor (EGF), a transforming growth factor-alpha (TGF-alpha), a basic fibroblast growth factor (bFGF), a brain-derived neurotrophic factor (BDNF), a hepatocyte growth factor (HGF), and a keratinocyte growth factor (KGF). Preferably, the receptor tyrosine kinase ligand may be an epidermal growth factor (EGF).

The receptor tyrosine kinase ligand may be treated at a concentration of, for example, 10 ng/ml or more; 20 ng/ml or more; 50 ng/ml or more; 75 ng/ml or more; 100 ng/ml or more; 120 ng/ml or more; 150 ng/ml or more; 200 ng/ml or more; 500 ng/ml or more; 1,000 ng/ml or more; 1,200 ng/ml or more; 1,500 ng/ml or more; 2,000 ng/ml or more; 5,000 ng/ml or more; 7,000 ng/ml or more; 10,000 ng/ml or more; or 15,000 ng/ml or more.

The combination of the above-described WNT/R-spondin activator, activator of the prostaglandin signaling pathway, and receptor tyrosine kinase ligand is preferably R-spondin 1, prostaglandin E2 (PGE2), and epidermal growth factor (EGF).

The combination of the WNT/R-spondin activator, the activator of the prostaglandin signaling pathway, and the receptor tyrosine kinase ligand is essential in terms of stemness maintenance, cell growth and proliferation, and has efficacy of enrichment culture and long-term culture of stem cells.

Although the present disclosure is not limited thereto, the WNT/R-spondin activator may exhibit an improved effect on stemness and/or cell proliferation, the activator of the prostaglandin signaling pathway may exhibit an improved effect on stemness and/or cell growth, and the receptor tyrosine kinase ligand may exhibit an improved effect on cell survival and/or cell proliferation.

In addition to the above-described essential composition, the culture medium of the present disclosure may further comprise any one or more selected from the group consisting of B27, N-acetyl-L-cysteine (NAC), nicotinamide, Gastrin, a TGF-beta inhibitor, a Wnt signaling pathway activator, a BMP inhibitor, and a p38 inhibitor for stable long-term subculturing.

The B27, the N-acetyl-L-cysteine (NAC), and the nicotinamide may be added as components of the basal medium, in particular, the B27 may be replaced by a generic formulation comprising one or more of components selected from the following list: biotin, cholesterol, linoleic acid, linolenic acid, progesterone, putrescine, retinyl acetate, sodium selenite, tri-iodothyronine (T3), DL-alphatocopherol (vitamin E), albumin, insulin, and transferrin.

The TGF-beta inhibitor may be any one selected from the group consisting of A-83-01, SB-431542, SB-505124, SB-525334, SD-208, LY-36494, and SJN-251, and preferably, A-83-01.

The Wnt signaling pathway activator may be any one selected from the group consisting of Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16, and preferably, Wnt3a.

The BMP inhibitor may be Noggin, Dorsomorphin, DMH1, or LDN-193189, and preferably, Noggin.

The p38 inhibitor may be any one selected from the group consisting of SB202190, SB203580, SB239063, SB706504, BIR796, JX401, EO1428, RWJ67657, SCIO469, VX745, TAK715, ML3403, DBM1285, and PH797804, and preferably, SB202190.

Each of these additional components may be adequately modulated in concentration within the general scope commonly used in culture medium.

In addition, in order to prevent loss of cells during subculture, a ROCK inhibitor, a Notch activator, or both of the ROCK inhibitor and the Notch activator may be further included in the culture medium at the initial stage of the culture, for example, immediately after the start of the culture, for 1 day, 2 days, 3 days, 4 days, or 5 days.

More specifically, at the initial stage of two-dimensionally culturing the single cell or the small cell clump in the culture medium comprising the WNT/R-spondin activator, the activator of the prostaglandin signaling pathway, and the receptor tyrosine kinase ligand, the culture medium may further include the ROCK inhibitor, the Notch activator, or all of the ROCK inhibitor and the Notch activator. This may be 1 day, 2 days, 3 days, 4 days, or 5 days immediately after the start of culture.

The Rho-associated protein kinase (ROCK) inhibitor serves to inhibit activity of a serine/threonine kinase acting as a target protein for Rho (Rho A, Rho B, and Rho C), and may be R-(+)-trans-4-(1-aminoethyl)-N-(4-pyridyl)cyclohexanecarboxamidedihydrochloridemonohydrate (Y-27632), 5-(1,4-diazepan-1-ylsulfonyl)isoquinoline (Fasudil or HA1077), and (S)-(+)-2-methyl 1-[(4-methyl-5-isoquinolinyl)sulfonyl]-hexahydro-1H-1,4-diazepindihydrochloride (H-1 152). More preferably, The ROCK inhibitor may be R-(+)-trans-4-(1-aminoethyl)-N-(4-pyridyl)cyclohexanecarboxamidedihydrochloridemonohydrate (Y-27632) .

The Y-27632 may be treated in an amount of, for example, about 0.1 uM to about 100 uM or about 0.1 uM to about 50 uM.

The Notch activator refers to a protein or small-molecule compound that activates a Notch pathway function, and may preferably be Jagged-1 (JAG 1).

The Jagged-1 (JAG 1) may be treated in an amount of, for example, about 0.1 uM to about 100 uM, or about 0.2 uM to about 50 uM, or about 0.3 uM to about 10 uM.

The culture of the intestinal stem cell population according to the present disclosure may be performed on feeder-cells or plates coated with extracellular matrix, as needed. The extracellular matrix is the same as described above.

The culture of the intestinal stem cell population according to the present disclosure may be performed 1 day, 2 days, 3 days, 4 days, 5 days, 6 days, 7 days, 8 days, 9 days, or 10 days, and 2 weeks, or 3 weeks or longer, but the present disclosure is not limited thereto, and subculture may be performed as needed.

The term "intestinal stem cell" herein refers to a cell that has the ability to replicate itself as an intestinal epithelial tissue-derived differentiated cell, and has the ability to differentiate into various types of specific cells present in intestinal epithelium. The intestinal stem cells may be differentiated into, for example, intestinal stem cells, intestinal progenitor cells, intestinal epithelial cells, intestinal goblet cells, intestinal endocrine cells, paneth cells, and the like.

The term "intestinal stem cell population" herein refers to a state in which intestinal stem cells are concentrated, and may be a tissue state, a cell cluster, or a single cell state.

The intestinal stem cell population of the present disclosure may exhibit an enhanced expression level for any one or more markers selected from the group consisting of LGR5, CD44, SOX9, LRIG1, LYZ, AXIN2, CTNNB, and MKI67. In addition, any one or more markers selected from the group consisting of LDHB, EIF3E, SOX9, and SHH may be expressed.

More specifically, the intestinal stem cell population may exhibit an enhanced expression level for LGR5, CD44, SOX9, LRIG1, LYZ, AXIN2, CTNNB, or MKI67, as compared to a control group cultured in a culture medium that does not include the WNT/R-spondin activator. The LGR5, CD44, SOX9, LRIG1, LYZ, AXIN2, CTNNB, or MKI67 is an intestinal stem cell marker, which means that as the enhanced expression level thereof is exhibited, a function of maintaining stem cell stemness and self-renewal is excellent.

Alternatively, the intestinal stem cells may express LDHB, EIF3E, SOX9, or SHH, which is a specific marker of intestinal stem cells and progenitor cells, by analyzing gene expression patterns by single cell RNA sequencing.

In the intestinal stem cell population of the present disclosure, 80, 81, 82, 83, 84, 85, 86, 87, 88, 88, 89, or 900 or more of cells constituting the population may be intestinal stem cells or progenitor cells. More specifically, the intestinal stem cell population may have 80, 81, 82, 83, 84, 85, 86, 87, 88, 88, 89, or 900 or more of cells including S phase cells, LGR5+ stem cells, and early enterocyte, based on the total number of cells in the population.

The S phase cell refers to a cell placed in S phase, which is a step in which DNA is replicated, among cells that undergo proliferation according to a cell cycle.

The LGR5+ stem cell refers to a stem cell expressing LGR5, which is an index indicating activity of adult stem cells, and the LGR5 refers to a target gene of a Wnt signal and a factor acting as a receptor of R-spondin that amplifies the Wnt signal.

The early enterocyte is a progenitor cell of epithelial cells surrounding the small and large intestines, and includes early enterocyte 1 and early enterocyte 2.

In addition, as needed, the early enterocyte may be used by freezing or thawing the cells under freezing conditions. Even under such long-term subculture conditions and/or freezing and thawing conditions, characteristics of the cells are maintained and the culture may proceed.

Accordingly, the present disclosure provides a method for cryopreserving an intestinal stem cell population, the method comprising the steps of: (a) dissociating a three-dimensional intestinal organoid derived from pluripotent stem cells into a single cell or a small cell clump;
(b) two-dimensionally culturing the single cell or the small cell clump in a culture medium comprising a WNT/R-spondin activator, an activator of a prostaglandin signaling pathway, and a receptor tyrosine kinase ligand to produce an intestinal stem cell population; and
(c) freezing the produced intestinal stem cell population.

The intestinal stem cell population produced through step (b) may be resuspended at a predetermined concentration (cell/mL) after centrifuging cells as needed.

A freezing medium may further include, for example, any one selected from the group consisting of dimethyl sulfoxide (DMSO); glucose; 1,2-propanediol; ethylene glycol; glycerol; formamide; ethanediol or butane-2,3 diol; hydroxyethyl starch (HES), dextran, sucrose, trehalose, lactose, raffinose, ribotol, mannitol, and polyvinylpyrrolidone (PVP).

Preferably, the cells are commercially sold or suspended in a freezing medium suitable for freezing and thawing of the cells. The freezing medium may be a Recovery^{™} Cell Culture Freezing Medium (Gibco) medium.

In some cases, the cell suspension medium for freezing is selected from a CHB medium, a CS10 medium, or a CS5 medium. The CHB medium is a cell suspension medium containing 500 (v/v) of fetal bovine serum (FBS), 400 (v/v) of a RPMI cell culture medium, and 100 (v/v) of dimethyl sulfoxide (DMSO). The CS10 medium (BioLife Solutions, Inc., Bothell, Washington) is a cell culture medium containing 100 (v/v) of DMSO and is essentially free of animal components or serum. The CS5 (BioLife Solutions, Inc.) medium is a cell culture medium comprising 5% (v/v) of DMSO and is essentially free of animal components or serum. The cells may be placed in vials and cryogenically frozen. A temperature sufficient to freeze the composition is about - 80°C to about -190°C.

The cryopreserved cells are thawed through a commonly known thawing method, as needed, and may be used as an intestinal stem cell population. In addition, the number of cells may be increased through subculturing, as needed.

The present disclosure also provides an intestinal stem cell population produced according to the culturing methods.

### Method for Producing Intestinal Epithelial Cells

The present disclosure provides a method for producing intestinal epithelial cells by culturing the intestinal stem cell population in a chemically defined differentiation medium through air-liquid interface culture.

The present disclosure provides a method for producing intestinal epithelial cells comprising a step of culturing an intestinal stem cell population in a differentiation medium comprising an activator of a prostaglandin signaling pathway, a receptor tyrosine kinase ligand, a p38 inhibitor, a WNT/R-spondin activator, and nicotinamide by air-liquid interface culture.

More specifically, there is provided a method for producing intestinal epithelial cells, the method comprising the steps of: (a) dissociating a pluripotent stem cell-derived three-dimensional intestinal organoid into a single cell or a small cell clump;
(b) two-dimensionally culturing the single cell or the small cell clump in a culture medium comprising a WNT/R-spondin activator, an activator of a prostaglandin signaling pathway, and a receptor tyrosine kinase ligand to produce an intestinal stem cell population; and
(c) culturing the intestinal stem cell population in a differentiation medium comprising an activator of a prostaglandin signaling pathway, a receptor tyrosine kinase ligand, a p38 inhibitor, a WNT/R-spondin activator, and nicotinamide by air-liquid interface culture.

The contents of the above-described pluripotent stem cells, organoids, and intestinal stem cells include the above-described descriptions, and redundant descriptions thereof will be omitted to avoid excessive complexity of the present specification.

In addition, since the culture medium of step (b) comprises the above-described WNT/R-spondin activator, activator of the prostaglandin signaling pathway, and receptor tyrosine kinase ligand, redundant descriptions thereof will be omitted to avoid excessive complexity of the present specification.

The term "differentiation medium" herein refers to a cell growth medium that allows undifferentiated stem cells to be generated as cells having some or all of characteristics of differentiated cells when cultured in the medium, and includes a basal medium.

The differentiation medium herein, which is optimized for production of intestinal epithelial cells, comprises an activator of a prostaglandin signaling pathway, a receptor tyrosine kinase ligand, a p38 inhibitor, a WNT/R-spondin activator, and nicotinamide.

The term "air-liquid interface culture" herein may refer to culturing in a culture vessel partially opened or a culture vessel partially filled with a medium, but the present disclosure is not limited thereto. For example, a surface of the cell or organoid may be exposed to air. The gas may be air and is not limited to a mixture of gas and composition found in the surrounding environment. Specifically, the present disclosure contemplates and encompasses gas mixtures having a composition different from the surrounding environment, for example, comprising a mixture enriched for a particular component or a mixture from which a particular component is depleted or removed.

When the cells are cultured at an air-liquid interface, the cells may make contact with air on an upper side of a porous substrate and make contact with a cell culture medium on a bottom side to culture the cells on the porous substrate. For example, a sufficient volume of medium may be added to a bottom of the culture vessel containing a porous substrate (for example, filter insert) such that the medium makes contact with a bottom surface of cells present on the porous substrate, but the cells are not encapsulated or submerged. The suitable porous substrates may be formed using any substance that does not adversely affect cell growth and differentiation. Exemplary porous substrate may be formed of a polymer such as polyethylene terephthalate (PET), polyester, or polycarbonate. The suitable porous substrate may be coated or uncoated. Examples of commercially available extracellular matrices include extracellular matrix proteins (Invitrogen) and basement membrane preparations (e.g., Cultrex (registered trademark) basal membrane extracts (Trevigen, Inc.), type I collagen (Invitrogen), or Vitrogel (registered trademark) (TheWell Bioscience Inc.) from Engelbreth-Holm-Swarm (EHS) murine sarcoma cells. Alternatively, the porous substrate may be coated with Matrigel (trademark) (BD Biosciences).

That is, the intestinal stem cells may be cultured in the presence of the transwell coated with the extracellular matrix. The extracellular matrix may be any of the extracellular matrices described above.

Preferably, the extracellular matrix may be Matrigel. A porosity of the substrate needs to be sufficient to maintain cell viability and promote cell differentiation.

The suitable substrate includes a filter insert having a porous size of about 0.3 to about 3.0 µm, about 0.3 to about 2.0 µm, about 0.3 to about 1.0 um, about 0.3 to about 0.8 um, about 0.3 to about 0.6 um, about 0.3 to about 0.5 um, about 0.5 to about 3.0 um, about 0.6 to about 3.0 um, about 0.8 to about 3.0 um, about 1.0 to about 3.0 um, about 2.0 um to about 3.0 um, and preferably about 0.4 um, and a pore density of about 50 million to about 120 million pores/cm², about 60 million to about 110 million pores/cm², about 70 million to about 100 million pores/cm², preferably about 80 million to about 100 million pores/cm², about 90 million to about 100 million pores/cm², and more preferably about 100 million pores/cm².

It may be advantageous to replace or regenerate the medium daily or every other day. The cells grown on a top of the porous substrate are generally not single cells, but rather the cells are in the form of sheets or present as aggregate clusters of cells. The cells cultured at the air-liquid interface may experience a much higher oxygen tension than the cells immersed in the medium.

The intestinal epithelial cells of the present disclosure may exhibit an enhanced expression level for at least one marker selected from the group consisting of VIL1, ECAD, FABP1, KRT20, LCT, LYZ, and MUC2.

More specifically, the intestinal epithelial cells may have characteristics in which the expression of at least one marker gene of LGR5, CD44, MKI67, SOX9, ASCL2, OLFM4, AXIN2, or CTNNB, which is an intestinal stem cell marker, is reduced, whereas the expression of VIL1, ECAD, FABP1, KRT20, LCT, LYZ, or MUC2, which is an intestinal epithelial cell marker, is increased.

In addition, the intestinal epithelial cells may exhibit an enhanced expression levels for AKR1B15, DHRS11, GALNT4, GALNT5, DHRS3, RDH10, AADAC, NR1I2, SULTE1, DOUX2, FABP1, SLC6A20, SLC43A1, and/or CLDN3 compared to the intestinal stem cells.

The intestinal epithelial cells may include small intestinal cells, mucus secretory cells, hormone secretory cells, and paneth cells.

The production of intestinal epithelial cells according to the present disclosure is not limited thereto, but may be performed 4 days, 5 days, 6 days, 7 days, 8 days, 9 days, or 10 days, and 2 weeks, 3 weeks or longer.

The present disclosure provides intestinal epithelial cells produced according to the method for producing intestinal epithelial cells.

### Use

### 1) Medicinal Use

The present disclosure provides a method for treating a patient suffering from intestinal diseases or at risk of developing intestinal diseases by utilizing intestinal stem cells or intestinal epithelial cells as a cell therapeutic agent.

The present disclosure provides a tissue therapeutic agent comprising an intestinal stem cell population and/or intestinal epithelial cells.

The present disclosure provides a pharmaceutical composition comprising an intestinal stem cell population and/or intestinal epithelial cells.

The present disclosure provides a pharmaceutical composition for preventing or treating intestinal diseases comprising an intestinal stem cell population and/or intestinal epithelial cells.

The present disclosure provides a pharmaceutical composition for assisting intestinal transplantation, which comprises an intestinal stem cell population and/or intestinal epithelial cells.

The present disclosure provides a pharmaceutical composition for preventing or treating intestinal diseases comprising: an intestinal stem cell population and/or intestinal epithelial cells; and intestinal organoids.

The intestinal stem cell population and/or intestinal epithelial cells may be used for therapeutic purposes as a biotechnological technology for restoring a function of cells or tissues.

For example, the therapeutic agent or pharmaceutical composition may be utilized as a transplant material and may be applied to treatment of various intestinal diseases. In particular, the use of the pharmaceutical composition as a material for regeneration and reconstruction of impaired (including dysfunction) intestinal tissue may be considered.

Accordingly, the pharmaceutical composition according to the present disclosure may be a tissue therapeutic agent.

Preferably, the cell may be an intestinal stem cell population.

In the present disclosure, the intestinal disease may be any one or more intestinal diseases selected from the group consisting of intestinal leakage syndrome, short bowel syndrome, irritable bowel syndrome, Crohn's disease, ulcerative colitis, intestinal Behcet's disease, infectious enteritis, ischemic bowel disease and radiation enteritis.

According to the present disclosure, the intestinal stem cell population may be used as a pharmaceutical composition for assisting intestinal transplantation of small intestinal organoids.

A prerequisite for transplantation efficiency and regenerative treatment efficiency is that blood is supplied to transplanted cells or tissues through blood vessel generation by rapidly engrafting on a transplanted site. Conventional small intestinal organoids or intestinal stem cells have a low engraftment rate during intestinal transplantation and have insufficient blood vessel formation, so that an initial transplantation efficiency is very low. The intestinal stem cell population according to the present disclosure may be used as an adjuvant for improving efficacy of intestinal transplantation by improving the engraftment rate and blood vessel formation efficiency during intestinal transplantation.

Further, the intestinal stem cell population of the present disclosure may be used as a composition for treating intestinal diseases, which includes an intestinal stem cell population and intestinal organoids.

In such transplantation, the intestinal stem cell population may be used in transplantation together with at least one biodegradable support selected from the group consisting of fibrin, laminin, collagen, gelatin, chitosan, alginate, hyaluronic acid, dextran, polylactic acid, poly(glycolic acid) (PGA), poly(lactic acid-co-glycolic acid) (poly(PLGA)), poly-ε-(caprolactone), polyanhydride, polyorthoester, polyvinyl alcohol, polyethylene glycol, polyurethane, polyacrylic acid, poly-N-isopropylacrylamide, poly(ethylene oxide)-poly(propylene oxide)-poly(ethylene oxide) copolymer, a copolymer thereof, and a mixture thereof.

That is, the cells serving as a target of gastric transplantation material may be used for transplantation as it is or by being embedded in the support described above.

In addition, dimethylsulfoxide (DMSO) and the like may be added for the purpose of protecting cells, an antibiotic substance and the like may be added for the purpose of blocking incorporation of bacteria, and various components (vitamins, cytokines, growth factors, steroids, and the like) may be added to the transplantation material of the present disclosure for the purpose of activating cells, proliferation, or inducing differentiation.

The transplantation material of the present disclosure is also available for the construction of an in vivo test system. For example, the above-described transplantation material may be transplanted into experimental animals such as mice, rats, guinea pigs, hamsters, pigs, cynomolgus monkeys, rhesus monkeys, chimpanzees, and the like, to produce humanized animals (human intestinal models). Such humanized animals are particularly useful for experiments such as pharmacokinetics and toxicity tests, and are expected to contribute to studies such as the effect of initial passing effects on oral drugs or pharmaceutical enteritis.

The term "prevention" herein means all actions that inhibit or delay the onset of intestinal diseases by administration of the composition, and the term "treatment" herein means all actions that improve or benefit the symptoms of intestinal diseases by administration of the composition.

The composition of the present disclosure may comprise 1.0 × 10⁵ to 1.0 × 10¹⁰, preferably, 1.0 × 10⁶ to 1.0 to 10⁹ cells per 1 ml.

The pharmaceutical composition of the present disclosure may be formulated into various formulations such as a liquid formulation, a suspension, and the like, according to the conventional method.

The pharmaceutical composition of the present disclosure may be administered while being formulated into a unit dosage form of a pharmaceutical formulation suitable for administration into the body of a patient according to the conventional method in the pharmaceutical field, and the formulation includes an effective dosage by one or several administrations. The preferred formulations suitable for this purpose include parenteral formulations such as injections, infusions, and transplants. In addition, the pharmaceutical composition may include a pharmaceutically acceptable common inert carrier and diluent. The pharmaceutically acceptable carrier and diluent may be biologically and physiologically friendly to the intestinal stem cell population and a recipient to be transplanted with the intestinal stem cell population. The diluent may include saline, a water-soluble buffer, a solvent and/or a dispersion medium, but the present disclosure is not limited thereto. In addition, for example, the injection may further include a preservative, a painless agent, a solubilizer, a stabilizer, or the like, and the preparation for topical administration may further include a base, an excipient, a lubricant, a preservative, or the like.

The composition of the present disclosure may be used unfrozen or frozen for later use. When the composition is to be frozen, a standard cryopreservative (e.g., DMSO, glycerol, and Epilife^{®} cell freezing medium (Cascade Biologics)) may be added to the cell population prior to freezing.

In addition, the composition may be transplanted and administered using an administration method commonly used in the art, and preferably, may be directly engrafted or transplanted into a disease site of the patient in need of treatment, but the present disclosure is not limited thereto. In addition, the administration may be performed by both non-surgical administration using a catheter and surgical administration such as injection or transplantation after incision in the disease site. The dosage may be 5 × 10⁵ to 10⁸/60 kg adults or 5 × 10⁵ to 10⁸/1 dose. However, it should be understood that an actual dosage of an active component should be determined in light of various relevant factors such as a disease to be treated, severity of the disease, a route of administration, a weight, age and sex of the patient, and the like, and thus the dosage does not limit the scope of the present disclosure in any way.

The present disclosure also provides a pharmaceutical composition comprising an intestinal stem cell population for use in prevention or treatment of intestinal diseases.

The present disclosure also provides a use of an intestinal stem cell population in production of a medicament for use in the prevention or treatment of intestinal diseases.

Provided is a method for treating intestinal diseases, which comprises a step of administering an intestinal stem cell population to a subject in need thereof.

The term "subject" refers to any animal that has been developed or may develop intestinal diseases, comprising humans, monkeys, cows, horses, sheep, pigs, chickens, turkeys, quail, cats, dogs, mice, mice, rabbits, or guinea pigs.

The present disclosure also provides a pharmaceutical composition comprising an intestinal stem population for use in assisting intestinal transplant.

The present disclosure also provides a use of an intestinal stem cell population in production of a medicament for use in assisting intestinal transplant.

Provided is a method for assisting intestinal transplantation, which comprises a step of administering an intestinal stem cell population to a subject in need thereof.

The present disclosure also provides a pharmaceutical composition comprising an intestinal stem population for use in assisting intestinal transplant.

The present disclosure also provides a use of an intestinal stem cell population in production of a medicament for use in assisting intestinal transplant.

Provided is a method for assisting intestinal transplantation, which comprises a step of administering an intestinal stem cell population to a subject in need thereof.

The present disclosure also provides a pharmaceutical composition comprising: an intestinal stem cell population; and a small intestinal organoid; for use in preventing or treating intestinal diseases.

The present disclosure also provides a use of an intestinal stem cell population and a small intestinal organoid in production of a medicament for use in preventing and treating intestinal diseases.

Provided is a method for treating intestinal diseases, which comprises a step of administering an intestinal stem cell population and a small intestinal organoid to a subject in need thereof.

### 2) Model

The present disclosure also provides an intestinal stem cell and/or intestinal epithelial cell model.

Specifically, the model may be used as a model by directly using intestinal stem cells or by using intestinal epithelial cells differentiated from the intestinal stem cells.

The intestinal stem cells according to the present disclosure may be used as a cell therapeutic agent for regenerative treatment of damaged intestinal tissue, and may help provide better results for research and clinical application of tissue regeneration. More specifically, since the intestinal stem cell according to the present disclosure is formed of a stem cell population that is engrafted into a tissue and plays a key role in tissue regeneration, the probability of engraftment into the damaged tissue is high and the damaged tissue may be rapidly repaired through direct regeneration.

In addition, the model may be used for various evaluations such as screening for discovering a therapeutic agent that is important to modulate regeneration of stem cells and the proliferation, and drug toxicity evaluation.

As needed, the intestinal stem cells of the present disclosure may also be used as a system model for development of intestinal stem cell lines, for example, stem cell lines through gene editing.

The term "stem cell line" refers to a cell that may be differentiated into other cell types which is completely differentiated to have specific specialized functions or cells that may be maintained in an undifferentiated state.

The intestinal stem cells according to the present disclosure may produce transformed cell lines by applying, for example, a gene editing technology using lentivirus or CRISPR-Cas9, and the transformed cell lines provide various research methods such as large-volume drug screening, efficacy evaluation, and real-time tracking of cell functions.

In addition, the intestinal stem cells may be labeled with any one fluorescent protein selected from the group consisting of a green fluorescence protein (GFP), a blue fluorescent protein (CFP), a yellow fluorescent protein (YFP), and a red fluorescent protein (DsRed), or may be used for analysis of characteristics of a target cell by transforming the fluorescent protein.

In particular, it is also possible to provide new cell lines that may be monitored in real time through transformation into the fluorescent protein or the like.

The present disclosure provides an intestinal epithelial cell model comprising the intestinal epithelial cells. Preferably, the intestinal epithelial model may be a small intestinal epithelial model.

In the intestinal epithelial cell model comprising intestinal epithelial cells differentiated from the intestinal stem cell population of the present disclosure, the intestinal epithelial cells may be intestinal epithelial cells having a crypt-villus structure. More preferably, the intestinal epithelial cells may be small intestinal epithelial cells. That is, the intestinal epithelial cell model may be an intestinal epithelial cell model having the crypt-villus structure.

The intestinal epithelial cell model may help provide more accurate results in study and clinical application with respect to bacterial or viral infection and intestinal drug metabolism. More specifically, the intestinal epithelial cell model not only has the shape and functional characteristics of a living tissue, but also has high stability and reproducibility, so that it is highly likely to be used for evaluating drug metabolism, bacterial or viral infection, microbial infection, and the like.

In other words, the intestinal epithelial cell model may be used as a platform for bacterial or viral infection, microbial infection, drug metabolism, and the like, so as to provide accurate prediction of symptom research, drug side effects, safety, and interaction with respect to bacterial or viral infection and microbial infection.

The intestinal epithelial cells produced according to the production method of the present disclosure or the intestinal epithelial cell model comprising the same may be used as an infectious disease model.

For example, the intestinal epithelial cells produced according to the production method of the present disclosure or the intestinal epithelial cell model comprising the same may be used as a bacterial or viral infectious disease model or a microbial infectious disease model. As for these bacteria, viruses, or microorganisms, any known bacteria, viruses, or microorganisms may be used. According to one embodiment of the present disclosure, the intestinal epithelial cells produced according to the production method of the present disclosure or the intestinal epithelial cell model comprising the same may be used as a SARS-CoV-2 virus infectious disease model.

The present disclosure provides a method for screening a therapeutic agent using an intestinal epithelial cell model according to the present disclosure.

Accordingly, the present disclosure provides a drug screening method comprising the steps of: (a) infecting an intestinal epithelial cell model with bacteria or viruses; (b) treating the infected intestinal epithelial cell model with a drug; and (c) confirming a reaction according to the drug treatment.

In addition, provided is a drug evaluation method comprising the steps of: (a) performing drug treatment on the intestinal epithelial cell model; and (b) evaluating absorbance or bioavailability of a drug in the intestinal epithelial cell model in (a).

In addition, provided is a method for providing an intestinal epithelial cell model for disease modeling, which comprises a step of infecting the intestinal epithelial cell model with bacteria or viruses.

In the present disclosure, a step of treating a test substance on the intestinal epithelial cell model may be performed. In the present disclosure, the test substance is a substance predicted to prevent, alleviate, or treat intestinal related diseases, and examples of a drug candidate substance, a test compound, or a test composition may include a small molecule drug, an antibody, an antisense nucleotide, short interfering RNA, short hairpin RNA, a nucleic acid, a protein, a peptide, other extracts, or a natural substance, but the present disclosure is not limited thereto. In the present disclosure, when expression of a biomarker protein for diseases or mRNA encoding the same is increased or reduced after treatment of the test substance as compared to before the treatment, a step of selecting the test substance as a therapeutic agent may be performed.

Examples of these diseases comprise bacterial or viral infectious diseases, microbial infectious diseases, or intestinal related diseases.

The term "bacterial or viral infectious disease or a microbial infectious disease" refers to any disease that may be caused by infection with a pathogenic bacterium or virus or microorganism.

In the present disclosure, the intestinal related diseases include inflammatory bowel disease (IBD), irritable bowel syndrome (IBS), Crohn's disease, ulcerative colitis (UC), short bowel syndrome, enterocolitis, Hirschsprung's disease, which is a genetic intestinal disease, and Celiac disease, but the present disclosure is not limited thereto.

Unless otherwise defined, all technical terms used in the present disclosure are used in the same meaning as commonly understood by those skilled in the art in the related field of the present disclosure. In addition, a preferred method or sample is described in the present specification, but those similar or equivalent thereto are also included in the scope of the present disclosure. The contents of all publications referred to herein as references are incorporated in the present disclosure.

### [Advantageous Effects]

Through the method for culturing intestinal stem cells according to the present disclosure, it is possible to easily and rapidly culture homogeneous intestinal stem cells. In addition, stable large-scale culture is possible through long-time subculturing and cryopreservation/thawing process, and the culture is possible while maintaining cell characteristics, thereby constructing a highly reproducible intestinal stem cell culture system.

The intestinal stem cell model produced through the culture system may be used as a new research model for human intestinal stem cell research, and particularly, intestinal stem cells derived from intestinal organoids differentiated from patient-specific induced pluripotent stem cells are a patient-specific cell source, and thus may be used as a cell source and a research model for the development of regenerative therapeutic agents for intestinal disease and the production of artificial organs.

In addition, the intestinal stem cells derived from the intestinal organoids are differentiated to intestinal epithelial cells through the method for producing intestinal epithelial cells according to the present disclosure, so that it is possible to secure a 2.5-dimensional intestinal epithelial cell model having diversity of intestinal cells and a structure similar to that of intestinal tissue.

The intestinal epithelial cell model, which is a model in which enzymes of nutrition and substance metabolism are increased, may perform absorption metabolism evaluation of various substances, and may serve as a barrier for dividing the outside and inside of the body. In addition, the intestinal epithelial cell model may be used for modeling various diseases including infectious diseases, and further, may be used in various fields such as therapeutic agent screening for treating diseases, and efficacy and toxicity evaluation of new drug candidate materials.

### [Description of Drawings]

FIG. 1 is a schematic diagram of a method for isolating an intestinal stem cell population from pluripotent stem cell-derived three-dimensional intestinal organoids to two-dimensionally culture the intestinal stem cell population.
FIG. 2 is a diagram showing a morphology of cells during culture of a two-dimensional intestinal stem cell population on a feeder and Matrigel.
FIG. 3 is a diagram showing a morphology of the intestinal stem cell population derived from embryonic stem cell-derived three-dimensional intestinal organoids (hESC-hIO) during two-dimensional culture on a feeder and Matrigel.
FIG. 4 is a diagram showing results of screening various types of extracellular matrix coating conditions in order to maximize engraftment of two-dimensional intestinal stem cell population.
FIG. 4(a) is a diagram showing a morphology of cells of the two-dimensional intestinal stem cell population when two-dimensional intestinal stem cells are cultured in culture vessels coated with 0.20 of gelatin, 10 µg/ml of Col Type I, 50 µg/ml of Col Type I, 1% of Matrigel, and 5% of Matrigel, respectively.
FIG. 4(b) is a graph showing results of calculating a surface area based on an area of cell colonies confirmed in FIG. 4(a) by using an Image J program.
FIG. 5 is a diagram showing screening results for finding an optimal culture medium composition for culturing the two-dimensional intestinal stem cell population.
FIG. 5(a) is a diagram in which a size of colonies of the intestinal stem cell population when a single factor is removed from components of the culture medium is confirmed through crystal violet (CV) staining.
FIG. 5(b) is a graph showing results of calculating a surface area based on a size of colonies confirmed through crystal violet (CV) staining in (a) by using the Image J program.
FIG. 6 is a diagram showing a morphology of the intestinal stem cell population in a culture medium for two-dimensionally culturing the two-dimensional intestinal stem cell population, when culture is performed in culture media from which essential factors (R-spondin 1, EGF, and PGE2) are removed, respectively.
FIG. 7 is a result showing that WNT3A and R-spondin 1 among the component factors of the culture medium serve as important functions in maintaining stemness and self-renewal of the two-dimensional intestinal stem cell population through activation of a WNT signal transduction system.
FIG. 7(a) is a graph in which it is confirmed through qPCR that expression of a marker gene, which is associated with the stemnness and self-renewal of the two-dimensional intestinal stem cell population, is reduced when the WNT signal transduction system is inhibited due to the removal of WNT3A and R-spondin 1.
FIG. 7(b) is a result confirmed through immunofluorescence staining of EdU and KI67 marker proteins that a growth ability of the two-dimensional intestinal stem cell population is reduced when the WNT signal transduction system is inhibited due to the removal of WNT3A and R-spondin 1.
FIG. 8 is a diagram showing a morphology of the two-dimensional intestinal stem cell population in which growth is inhibited when the WNT signal transduction system is inhibited due to removal of a WNT ligand (WNT3A or R-spondin 1) or treatment with a WNT inhibitor (WNT-C59 or XAV939) .
FIG. 9 is a diagram showing a morphology of the two-dimensional intestinal stem cell population in which cell proliferation is inhibited when an EGFR signal transduction system is inhibited due to removal of an EGF ligand or EGF inhibitor (PD0325901) treatment, and results of cell survival/death (Calcein-AM (live)/Etidium homodimer1 (dead)) assay.
FIG. 10 is a result showing that growth of the two-dimensional intestinal stem cell population is inhibited when a PGE2 signal transduction system is inhibited due to removal of a PGE2 ligand or treatment with the PGE2 inhibitor (EP2i or EP4i) .
FIG. 10(a) is a diagram showing a morphology of the two-dimensional intestinal stem cell population in which growth is inhibited when the PGE2 signal transduction system is inhibited due to the removal of PGE2 ligand or the treatment with the PGE2 inhibitor (EP2i or EP4i) .
FIG. 10(b) is a graph showing that PGE2 and PTGER4 are specifically expressed in two-dimensional intestinal stem cells among the receptors of PTGER2 (PTGER1 to PTGER4) and PGE2 synthase (PTGES).
FIG. 11 is a result showing that stable long-term culture is possible through subculture of the two-dimensional intestinal stem cell population.
FIG. 11(a) is a diagram showing a morphology of the two-dimensional intestinal stem cell population of P0, P1, P3, P5, P10, P20, and P30.
FIG. 11(b) is a graph showing an increase in the total number of cells during a subculture process.
FIG. 12 is a diagram in which when other factors except the culture medium essential factors (R-spondin 1, EGF, and PGE2) of FIG. 6 are removed from the culture media, respectively, the factors do not affect the growth of the two-dimensional intestinal stem cells of P0, but cell engraftment and proliferation ability are reduced during subculture.
FIG. 13 is a diagram showing that cell engraftment increases by treatment with a NOTCH activator (Jagged-1 or Valproic acid) or a ROCK inhibitor (Y-27632) during subculture of two-dimensional intestinal stem cells.
FIG. 13(a) is a diagram showing a morphology of an engrafted two-dimensional intestinal stem cell population when the NOTCH activator and the ROCK inhibitor are treated alone or simultaneously.
FIG. 13 (b) is a graph showing results of measuring the number of cells based on the two-dimensional intestinal stem cell population engrafted in FIG. 13(a) by using a Countess III cell counter.
FIG. 14 is a diagram showing morphological characteristics of cells when the two-dimensional intestinal stem cell population is cultured under optimized culture conditions.
FIG. 14(a) is a diagram showing that the two-dimensional intestinal stem cell population cultured under optimized culture conditions is formed of a single layer.
FIG. 14(b) is a diagram showing 100% survival rate of the two-dimensional intestinal stem cell population cultured under the optimized culture conditions.
FIG. 15 is a diagram showing that cryopreservation and thawing of the two-dimensional intestinal stem cells are possible under the optimized culture conditions and highly reproducible culture with little difference between batches is possible.
FIG. 15(a) is a diagram showing a morphology of cells at 2, 4, and 8 days after culturing the thawed two-dimensional intestinal stem cell population after cryopreservation.
FIG. 15(b) is a diagram showing that the two-dimensional intestinal stem cell population may be cultured for a long time with little difference between batches.
FIG. 16 is a schematic diagram of an analysis process for analyzing a cell composition and characteristics of the two-dimensional intestinal stem cell population through single cell RNA sequencing (scRNA-seq).
FIG. 17 is a diagram showing that the two-dimensional intestinal stem cell population includes a large amount of cells exhibiting characteristics of epithelial cells compared to mesenchymal stromal cells through analysis of marker gene expression of the epithelial cells and the mesenchymal stromal cells.
FIG. 18 is a diagram showing characteristics similar to those of intestinal epithelial cells of a fetus at 6 to 8 weeks as a result of analyzing a gene of the two-dimensional intestinal stem cell population.
FIG. 18(a) is a diagram showing, in a heatmap, results of comparing expression of marker genes of the two-dimensional intestinal stem cell population and human intestinal epithelial cells from a fetus to an adult.
FIG. 18(b) is a graph showing, in a dendrogram, results of hierarchical clustering of the results of FIG. 18(a) according to similarity of a marker gene expression pattern.
FIG. 19 is a diagram showing types and composition of cells constituting the two-dimensional intestinal stem cell population based on analysis results of a single cell transcript.
FIG. 19(a) is a diagram showing, on an UMAP, types and distribution of cells constituting the two-dimensional intestinal stem cell population based on the analysis results of the single cell transcript.
FIG. 19 (b) is a table showing a percentage of cells constituting the two-dimensional intestinal stem cell population based on the results of FIG. 19(a).
FIG. 20 is a diagram showing results of comparing the analysis results of the single cell transcript of the two-dimensional intestinal stem cell population with the analysis results of the single cell transcript of human intestinal epithelium disclosed in the reference.
FIG. 20(a) is a diagram showing, on a UMAP, types and distribution of cells constituting human intestinal epithelium through analysis of the single cell transcript reported in the reference.
FIG. 20(b) is a diagram showing that the cell composition of the two-dimensional intestinal stem cell population is mainly composed of intestinal epithelial stem cells and progenitor cells, in which the analysis result of the single cell transcript of the two-dimensional intestinal stem cell population is shown on the same UMAP together with the results of FIG. 20(a).
FIG. 20(c) is a diagram showing types and expression patterns of marker genes specifically expressed for each type of cells constituting human intestinal epithelium through the analysis of the single cell transcript.
FIG. 21 is a diagram showing that the two-dimensional intestinal stem cell population includes a plurality of cells exhibiting characteristics of stem cells or progenitor cells through analysis of the expression patterns of marker genes that specifically express stem cells.
FIG. 22 is a diagram showing verification results through immunofluorescence staining in order to confirm that the two-dimensional intestinal stem cell population is mainly composed of cells exhibiting characteristics of stem cells and progenitor cells.
FIG. 22(a) is a diagram showing results of confirming that most of the cells of the two-dimensional intestinal stem cell population are stem cells or progenitor cells through immunofluorescence staining of LDHB, EIF3E, SOX9, and KI67, which are stem cell-specific marker proteins.
FIG. 22(b) is a diagram showing results of confirming that the two-dimensional stem cell population is not differentiated to secretory cells through the fact that expressions of MUC2 and CHGA, which are marker proteins of the secretory cells among differentiated cells constituting intestinal epithelium, are not observed at all.
FIG. 23 is a schematic diagram of a method for differentiating a two-dimensional intestinal stem cell population to 2.5-dimensional intestinal epithelial cells through air-liquid interface culture.
FIG. 24 is a diagram showing a morphology of cells when the two-dimensional intestinal stem cell population is differentiated after removing a single factor from a culture medium in order to find essential factors important for differentiation to 2.5-dimensional intestinal epithelial cells through air-liquid interface culture.
FIG. 25 is a diagram showing that the two-dimensional intestinal stem cell population is normally differentiated into 2.5-dimensional intestinal epithelial cells through air-liquid interface culture in a minimal medium composed of essential factors.
FIG. 25(a) is a diagram showing that when the two-dimensional intestinal stem cell population is differentiated into 2.5-dimensional intestinal epithelial cells through air-liquid interface culture in a medium (Full M) for culturing a two-dimensional intestinal stem cell population and a minimal medium (minimal M) composed of essential factors, the entire population is normally differentiated.
FIG. 25(b) is a diagram showing that various types of pluripotent stem cell-derived two-dimensional intestinal stem cell population is differentiated into 2.5-dimensional intestinal epithelial cells through air-liquid interface culture in the minimal medium.
FIG. 25(c) is a diagram showing that the 2.5-dimensional intestinal epithelial cells differentiated through air-liquid interface culture may be cultured so as to have high reproducibility with little difference between batches.
FIG. 26 is a graph showing that, when differentiation of the two-dimensional intestinal stem cell population into 2.5-dimensional intestinal epithelial cells is performed through air-liquid interface culture, expression of differentiated cell marker genes gradually increases as the differentiation progresses.
FIG. 27 is a diagram showing that a villus-like structure is formed and expression of the differentiated cell marker protein increases when the two-dimensional intestinal stem cell population is differentiated to 2.5-dimensional intestinal epithelial cells through air-liquid interface culture.
FIG. 27 (a) is a diagram in which a cross-section of cells is confirmed through H&E staining at 4 days, 8 days, and 12 days and an expression level of the marker protein is confirmed through immunofluorescence staining after the differentiation of the two-dimensional intestinal stem cell population into 2.5-dimensional intestinal epithelial cells through air-liquid interface culture.
FIG. 27(b) is a graph showing results of measuring a thickness of the cross-section of the cells confirmed through the H&E staining in FIG. 27(a) by using the Image J program.
FIG. 27(c) is a graph showing that, after the differentiation of the two-dimensional intestinal stem cell population into 2.5-dimensional intestinal epithelial cells is performed through air-liquid interface culture, a barrier function of intestinal epithelial cells at 4, 8, and 12 days is confirmed through measurement of transepithelial electrical resistance (TEER).
FIG. 28 is a diagram showing results of confirming the two-dimensional intestinal stem cell population and 2.5-dimensional intestinal epithelial cells, which are differentiated through air-liquid interface culture, through principle component analysis (PCA) using transcript expression patterns, in order for comparative analysis with pluripotent stem cells (hPSC), immature three-dimensional intestinal organoid (Control hIO), mature three-dimensional intestinal organoid (Mature hIO), functional intestinal epithelial cells (hIEC), and human intestinal epithelial tissue (hSI).
FIG. 29 is a diagram showing a gene cluster and a representative gene showing a difference in expression pattern when the differentiation to intestinal epithelial cells is performed through transcript analysis of the two-dimensional intestinal stem cell population and 2.5-dimensional intestinal epithelial cells differentiated through air-liquid interface culture.
FIG. 29(a) is a table showing types of gene clusters whose expression patterns increase or decrease when the differentiation of the two-dimensional intestinal stem cell population into 2.5-dimensional intestinal epithelial cells is performed through air-liquid interface culture.
FIG. 29(b) is a graph showing results of verifying expression patterns of representative genes of the gene clusters found in FIG. 29(a) through a qPCR experiment.
FIG. 30 is a schematic diagram for a process of producing an intestinal stem cell line expressing a fluorescent protein through gene delivery using lentivirus.
FIG. 31 is a diagram showing a morphology and a fluorescence protein expression pattern for each step of production of the intestinal stem cell line expressing a fluorescence protein.

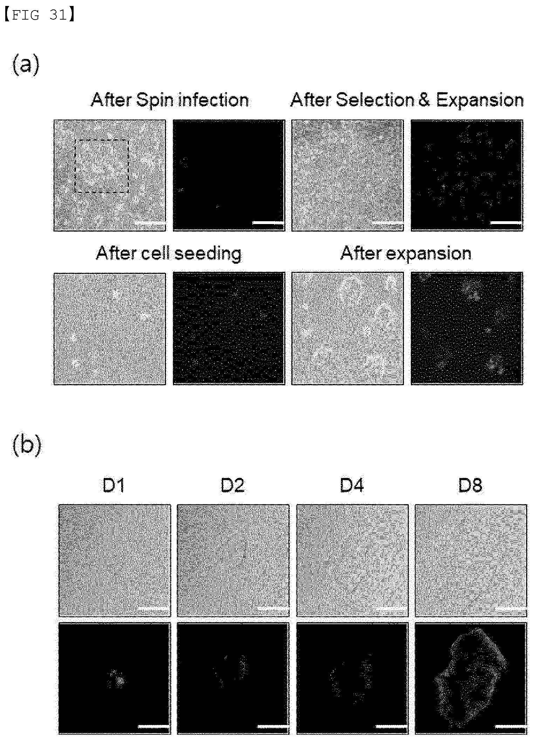

FIG. 31(a) is a diagram showing morphologies and fluorescence protein expression patterns of intestinal stem cell line, in which immediately after fluorescence genes are transported using a lentivirus (after spin infection), clone is selected using antibiotics (after selection and expansion), after the selection, isolation into single cells is performed using trypsin (trypsin-EDTA) in order to produce a single cell-derived cell line, and immediately after the subculturing (after cell seeding), and after culturing is performed such that the intestinal stem cell line has a certain size or greater (after expansion).
FIG. 31 (b) is a diagram showing, by date, morphologies and fluorescence protein expression patterns of cells that are cultured by isolating a single cell-derived fluorescence protein expression clone produced in FIG. 31(a) by treatment with collagenase type IV+Dispase, and then isolating only the single clone.
FIG. 32 is a diagram showing morphologies and fluorescence protein expression patterns of cells after the differentiation of a three-dimensional intestinal organoid into 2.5-dimensional intestinal epithelial cells through air-liquid interface culture by using the intestinal stem cell line produced in FIG. 31.
FIG. 32(a) is a diagram showing morphologies and fluorescence protein expression patterns of cells at 6, 14, and 20 days after the intestinal stem cell line expressing the fluorescence protein is differentiated into the three-dimensional intestinal organoids in a Matrigel dome.
FIG. 32(b) is a diagram showing morphologies and fluorescence protein expression patterns of intestinal epithelial cells on day 8 after the differentiation of intestinal stem cell lines expressing the fluorescence protein into 2.5-dimensional intestinal epithelial cells is performed through air-liquid interface culture.
FIG. 33 is a schematic diagram illustrating the processes for modeling intestinal diseases and transplanting intestinal stem cells. This figure demonstrates the confirmation of regenerative treatment effects using two-dimensional intestinal stem cell population and their applicability as cell therapeutic agents.
FIG. 34 is a diagram depicting the process of transplanting the two-dimensional intestinal stem cell population using a murine colonoscopy and the condition of the mice post-transplantation.
FIG. 34(a) is a diagram showing a photograph (left) of a murine colonoscopy device capable of injecting cells and an image (right) of the two-dimensional intestinal stem cell population being transplanted, captured using the murine colonoscopy.
FIG. 34(b) is a diagram showing the condition of the mice immediately after the transplantation of the two-dimensional intestinal stem cell population.
FIG. 35 is a diagram illustrating the changes in body weight over time following the transplantation of Matrigel or two-dimensional intestinal stem cells into mice.
FIG. 36 includes a chart and a graph illustrating the post-transplantation survival rates of mice transplanted with either Matrigel or two-dimensional intestinal stem cells.
FIG. 37 is a diagram showing the results of confirming the regeneration effect of intestinal epithelium using murine colonoscopy at 3 days and 14 days post-transplantation of Matrigel or two-dimensional intestinal stem cells into mice with intestinal damage. This evaluation is performed before and after intestinal epithelium damage modeling using Hot-EDTA.
FIG. 38 is a diagram showing the engraftment of the two-dimensional intestinal stem cell population in the damaged intestinal epithelium.
FIG. 38(a) is a diagram illustrating the engraftment of the two-dimensional intestinal stem cell population labeled with DiR fluorescent dye at the damaged intestinal site, as observed 14 days post-transplantation using IVIS equipment.
FIG. 38(b) is a diagram demonstrating the normal engraftment of green fluorescent protein (GFP)-expressing intestinal stem cell lines in the damaged intestinal epithelium. This was confirmed by transplanting the intestinal stem cell lines, isolating the intestines after 14 days, and verifying the expression of the fluorescent protein.
FIG. 39 is a diagram demonstrating the regenerative ability of the two-dimensional intestinal stem cell population, showing normal regeneration of damaged intestinal epithelium following transplantation of the population into the damaged site.
FIG. 39(a) is a diagram illustrating that intestinal epithelial tissue regeneration occurs only when the two-dimensional intestinal stem cell population is transplanted. This is shown through H&E and AB-PAS staining analysis of the intestinal epithelial tissue morphology after transplantation of either Matrigel or the two-dimensional intestinal stem cell population into the damaged site, followed by isolation of the intestinal tissue after 14 days.
FIG. 39(b) is a diagram showing the results of confirming murine intestinal regeneration by human intestinal stem cells, indicated by human-specific antibody expression throughout the entire crypt-villus structure. This was verified by transplanting the intestinal stem cells into the damaged intestinal site and isolating the intestines after 14 days to confirm human-specific cytokeratin expression.
FIG. 40 is a schematic diagram for the entire process of modeling diseases by infecting SARS-CoV-2 virus into 2.5-dimensional intestinal epithelial cells differentiated from immature or mature two-dimensional intestinal stem cell population through air-liquid interface culture.
FIG. 41 is a diagram showing results of observing a morphology of 2.5-dimensional intestinal epithelial cells differentiated from the immature or mature two-dimensional intestinal stem cell population through air-liquid interface culture at 2 days, 4 days, 6 days, 8 days, and 10 days after the start of differentiation.
FIG. 42 is a graph showing that the intestinal epithelial cells differentiated from the mature two-dimensional intestinal stem cell population has expression of marker genes indicating intestinal maturity, which is relatively higher than expression of marker genes of the intestinal epithelial cells differentiated from the immature two-dimensional intestinal stem cell population.
FIG. 43 is a diagram showing results of confirming that the intestinal epithelial cells differentiated from the mature two-dimensional intestinal stem cell population has expression of receptors important for SARS-CoV-2 infection, which is relatively higher than expression of receptors of the intestinal epithelial cells differentiated from the immature two-dimensional intestinal stem cell population.
FIG. 43(a) is a graph showing results of analyzing only the expression of receptors important for SARS-CoV-2 infection in the results of analyzing a transcript of intestinal epithelial cells differentiated from the mature or immature two-dimensional intestinal stem cell population.
FIG. 43(b) is a diagram showing results of confirming, using immunofluorescence staining, an expression level of an ACE2 protein which is confirmed that the expression of the ACE2 protein increases in FIG. 43(a) among the receptors important for SARS-CoV-2 infection in the intestinal epithelial cells differentiated from the mature or immature two-dimensional intestinal stem cell population.
FIG. 44 is a graph showing results of confirming a transcript of a virus using qPCR in the intestinal epithelial cells, in which the intestinal epithelial cells differentiated from the mature two-dimensional intestinal stem cell population experience a level of SARS-CoV-2 infection higher than a level of SARS-CoV-2 infection of the intestinal epithelial cells differentiated from the immature two-dimensional intestinal stem cell population.

### [Mode for Carrying out the Invention]

Hereinafter, the present disclosure will be described in more detail with reference to the examples. The examples are intended to describe the present disclosure in more detail, and the scope of the present disclosure is not limited to these embodiments.

### Experimental Example 1. Cell Culture and iPSC Production

hESCs (human embryonic stem cells) and hPSCs (human pluripotent stem cells) including hiPSCs (human induced pluripotent stem cells) were cultured by a known method (Molecular carcinogenesis 55, 387-396 (2016), Proteomics 15, 2220-2229 (2015)). Non-insertable-hiPSCs were transfected by electroporation and reprogrammed using Episomal iPSC reprogramming vector (Cat. No. A14703. Invitrogen, Carlsbad, CA, USA) according to the known method.

After 5 days of electroporation, fibroblasts were plated in 1 x 10⁵/well on Matrigel (BD Biosciences, San Diego, CA, USA)-coated 6-well plates, and cultured in E8 medium (Stem Cell Technologies, Vancouver, Canada). After 3 weeks, hiPSC colonies were selected, and the cell number was increased for subculture and subsequent characterization.

### Experimental Example 2. Differentiation of hPSCs into Intestinal Organoids (hIOs) for Production of Three-Dimensional Intestinal Organoids

Human intestinal organoids (hIOs) were prepared using a known method (Nature 470, 105-109 (2011)). To induce definitive endoderm, hPSCs were plated on Matrigel or dishes coated with ECMatrix^{™}-511 and treated with 100 ng/ml of Activin A (R&D Systems, Minneapolis, MN, USA) in an RPMI 1640 medium having purified fetal bovine serum (dFBS, HyClone, Thermo Fisher Scientific Inc., Waltham, MA, USA) at concentrations of 00, 0.2% and 2% for 3 days. In addition, in order to differentiate the hPSCs to 3D hindgut spheroids, 500 ng/ml of FGF4 (R&D Systems) and 3 µM of CHIR99021 (TOCRIS) were treated together with the RPMI 1640 medium comprising 2% of dFBS for 4 to 6 days. From day 4, which was induced to the hindgut, spheroids were inserted into Matrigel (BD Biosciences), cultured in a hIO medium (2 mM of L-glutamine, 1% of penicillin-streptomycin, and 15 mM of HEPES buffer in Advanced DMEM F12) comprising 1X B27 (Invitrogen), 200 to 250 ng/ml of R-spondin 1 (R&D Systems), 100 ng/ml of EGF (R&D Systems), and 40 to 50 ng/ml of Noggin (R&D Systems), and subcultured every 10 to 14 days. For maturation of intestinal organoids, 1 ng/ml of interleukin 2 (IL-2, R&D Systems) was added to the hIO medium for about 2 passage and cultured.

### Experimental Example 3. Method for Isolating and Culturing Intestinal Stem Cells from Three-Dimensional Intestinal Organoids

The three-dimensional intestinal organoids were isolated from a Matrigel dome and the remaining three-dimensional intestinal organoids were removed as much as possible by pipetting. The separated organoids were placed in 1 ml of 0.25% trypsin-EDTA (TE, Invitrogen) and incubated at 37°C a water bath for about 5 minutes. Then, pipetting was performed less than five times so that the organoids were allowed to be isolated into single cells and small clumps, and a basal medium was added so that the total volume thereof was 10 ml. The cells fed into a centrifuge were placed on culture dishes coated with feeder cells or 1% of Matrigel (Corning). Thereafter, 200 ng/ml of R-spondin 1 (R&D Systems), 100 ng/ml of EGF (R&D Systems), and 2.5 µM of Postaglandin E2 (Sigma-aldrich) were used as main components, cultured in an intestinal stem cell culture medium (2 mM of L-glutamine, 1% of Penicillin-Streptomycin, and 15 mM of HEPES buffer in Advanced DMEM F12) comprising, as an auxiliary component, one or more selected from the group consisting of 1X B27 (Invitrogen), 80 ng/ml of Noggin (R&D Systems), 10 nM of [Leu15]-Gastrin I (Sigma-aldrich), 100 ng/ml of human recombinant WNT3A (R&D Systems), 500 nM of A-83-01 (Tocris), 10 uM of SB202190 (Sigma-aldrich), 1 mM of N-acetylcysteine (Sigma-aldrich), and 10 mM of nicotinamide (Sigma-aldrich), and subcultured every 7 to 10 days. During the first 2 days of subculture, 1 µM of Jagged-1 (Anaspec) and/or 2.5 µM of Y-27632 (Tocirs) were added to the intestinal stem cell culture medium.

### Experimental Example 4. Freezing and Thawing of Intestinal Stem Cells

For freezing the intestinal stem cells, the intestinal stem cells were washed 1 to 2 times with PBS at 3 to 5 days after subculture, and treated with TE at 37°C for 5 minutes in the same manner as subculture to isolate the intestinal stem cells into single cells or small cell clumps, and then washed once with a hIO medium (2 mM of L-glutamine, 1% of Penicillin-Streptomycin, and 15 mM of a HEPES buffer in Advanced DMEM F12). Thereafter, the cells were well-suspended by adding a freezing medium (Recovery^{™} Cell Culture Freezing Medium, Gibco), and then the resulting suspension was frozen and stored in an LN2 Tank for a long period of time.

A warm (37°C) medium was prepared in advance for thawing frozen intestinal stem cells. The frozen cells were rapidly thawed, washed once with an intestinal stem cell culture medium, and cultured in an intestinal stem cell culture medium comprising a WNT/R-spondin activator, a prostaglandin signaling activator, and a receptor tyrosine kinase ligand on a culture dish coated with Matrigel. The culture medium of the intestinal stem cells was changed once every 2 days, and subculture was performed in the same manner after culturing for 3 to 7 days depending on a state of the cells at the time of initial thawing.

### Experimental Example 5. Method for Differentiating Intestinal Epithelial Cells Using Air-Liquid Interface Culture

The intestinal stem cells grown to a density of 70 to 800 were washed 1-2 times with PBS, and then treated with TE in an incubator at 37°C for 5 to 7 minutes. The intestinal stem cells isolated into single cells were collected and then diluted using the hIO medium. The cells were gathered by centrifugation, supernatant was removed, the intestinal stem cell culture medium was added thereto and sufficiently mixed, and then the number of cells was measured using Countess III cell counter (Thermo Scientific. Inc.). 2.5-3.5 X 10⁵ cells were placed in an insert of 12-transwell plate (Corning) coated with 1% of Matrigel, and cultured in an incubator. After the cell density reached 1000, the culture medium of an upper layer was completely removed, and the culture medium of a lower layer was replaced with a differentiation medium (2 mM of L-glutamine, 1% of Penicillin-Streptomycin, and 15 mM of a HEPES buffer in Advanced DMEM F12) comprising 200 ng/ml of R-spondin 1 (R&D Systems), 100 ng/ml of EGF (R&D Systems), 2.5 µM of Prostaglandin E2 (Sigma-aldrich), 10 µM of SB202190 (Sigma-aldrich), and 10 mM of nicotinamide (Sigma-aldrich). Thereafter, once every 2 days, a surface of the upper layer was washed with the PBS or hIO medium, and the lower layer was replaced with a new differentiation medium and cultured for 8 to 12 days.

### Experimental Example 6. Method for Measuring Cell Viability

To measure viability of a two-dimensional intestinal stem cell population cultured in a culture dish coated with 1% of Matrigel, a kit (LIVE/DEAD Viability/Cytotoxicity Kit, Invitrogen) capable of distinguishing and staining surviving cells and dead cells was used. The surviving cells were stained with calcein-AM, and the dead cells were stained with ethidium homodimer-1. The stained cells were observed through a fluorescence microscope (Olympus).

### Experimental Example 7. Method for Measuring Cell Growth Rate

The two-dimensional intestinal stem cell population was plated on a culture dish coated with 1% of Matrigel and then cultured for 2 to 7 days. Thereafter, the culture medium was removed, and washing was performed 1 to 2 times with PBS (Sigma-aldrich), and TE (Invitrogen) was added thereto to detach the cells. After centrifugation, a medium was added to isolate the cells into single cells, and then the number of cells was measured using CountessIII cell counter (Thermo Scientific. Inc.).

### Experimental Example 8. Crystal Violet (CV) Staining

The intestinal stem cell population was fixed with 4% of paraformaldehyde (PFA) and stained with 0.02% of crystal violet solution (Sigma-aldrich) at room temperature for 10 minutes. Thereafter, images were acquired after washing three times with sterile water. A colony size of the intestinal stem cell population was analyzed using Image J software (National Institute of Health).

### Experimental Example 9. Quantitative Real-Time RT-PCR (qRT-PCR)

Total RNA was extracted from the cells using RNeasy kit (Qiagen) and reverse transcribed using a Superscript III cDNA synthesis kit (Invitrogen). qRT-PCR was performed in a 7500 Fast Real-time PCR system (Applied Biosystems, Foster City, CA, USA) by a known method (Cho et al., Oncotarget 6, 23837-23844, 2015). All experiments were repeated three times, and a CT value of each target gene was calculated using software provided by the manufacturer. The base sequences of the used primers are shown in Table 1.

**[Table 1]**

| Target gene | Primer (Forward) | Primer (Reverse) |
|---|---|---|
| *GAPDH* | GAAGGTGAAGGTCGGAGTC (SEQ ID NO. 1) | GAAGATGGTGATGGGATTTC (SEQ ID NO: 2) |
| *LGR5* | TGCTCTTCACCAACTGCATC (SEQ ID NO: 3) | CTCAGGCTCACCAGATCCTC (SEQ ID NO: 4) |
| *CD44* | CCAGAAGGAACAGTGGTTTGGC (SEQ ID NO: 5) | ACTGTCCTCTGGGCTTGGTGTT (SEQ ID NO: 6) |
| *SOX9* | GTACCCGCACTTGCACAAC (SEQ | TCTCGCTCTCGTTCAGAAGTC (SEQ ID |
| | ID NO: 7) | NO: 8) |
| *LRIG1* | GACCCTTTCTGACCGACAA (SEQ ID NO: 9) | CGCTTTCCACGGCTCTTT (SEQ ID NO: 10) |
| *LYZ* | AAAACCCCAGGAGCAGTTAAT (SEQ ID NO: 11) | CAACCCTCTTTGCACAAGCT (SEQ ID NO: 12) |
| *MKI67* | TGACCCTGATGAGAAAGCTCAA (SEQ ID NO: 13) | CCCTGAGCAACACTGTCTTTT (SEQ ID NO: 14) |
| *AXIN2* | GAGTGGACTTGTGCCGACTTCA (SEQ ID NO: 15) | GGTGGCTGGTGCAAAGACATAG (SEQ ID NO: 16) |
| *CTNNB* | TCTGAGGACAAGCCACAAGATTACA (SEQ ID NO: 17) | TGGGCACCAATATCAAGTCCAA (SEQ ID NO: 18) |
| *ASCL2* | CGTGAAGCTGGTGAACTTGG (SEQ ID NO: 19) | GGATGTACTCCACGGCTGAG (SEQ ID NO: 20) |
| *OLFM4* | ACCTTTCCCGTGGACAGAGT (SEQ ID NO: 21) | TGGACATATTCCCTCACTTTGGA (SEQ ID NO: 22) |
| *VIL1* | AGCCAGATCACTGCTGAGGT (SEQ ID NO: 23) | TGGACAGGTGTTCCTCCTTC (SEQ ID NO: 24) |
| *ECAD* | GTCACTGACACCAACGATAATCCT (SEQ ID NO: 25) | TTTCAGTGTGGTGATTACGACGTTA (SEQ ID NO: 26) |
| *FABP1* | GGAGGAATGTGAGCTGGAGACA (SEQ ID NO: 27) | TATGTCGCCGTTGAGTTCGGTC (SEQ ID NO: 28) |
| *KRT20* | TGGCCTACACAAGCATCTGG (SEQ ID NO: 29) | TAACTGGCTGCTGTAACGGG (SEQ ID NO: 30) |
| *LCT* | GGCAGTCTGGGAGTTTTAGG (SEQ ID NO: 31) | ATGCCAAAATGAGGCAAGTC (SEQ ID NO: 32) |
| *MUC2* | TGTAGGCATCGCTCTTCTCA (SEQ ID NO: 33) | GACACCATCTACCTCACCCG (SEQ ID NO: 34) |
| *CHGA* | TGACCTCAACGATGCATTTC (SEQ ID NO: 35) | CTGTCCTGGCTCTTCTGCTC (SEQ ID NO: 36) |
| *AKR1B15* | GAGGACCTGTTCATCGTCAGCA (SEQ ID NO: 37) | CGTCCAGATAGCTCAGCTTCAG (SEQ ID NO: 38) |
| *DHRS11* | CACCAGTGGTTGGAAGGACATG (SEQ ID NO: 39) | CATCGTCCACATTCCGCTCCTT (SEQ ID NO: 40) |
| *GALNT4* | GTCAAGAAGGCTCTCAGACCTC (SEQ ID NO: 41) | GTTCATCCTCGTTGAGCTGGAG (SEQ ID NO: 42) |
| *GALNT5* | CCAGTGGATAGAGCCATTGAAGA (SEQ ID NO: 43) | TCTCAGGAGAGTGGACCACACT (SEQ ID NO: 44) |
| *DHRS3* | GGGCACTGAGTGCCATTACTTC (SEQ ID NO: 45) | CGGCATTGTTCACCAGGATGGT (SEQ ID NO: 46) |
| *RDH10* | GGCATCACCTTCTGGAATGTCC (SEQ ID NO: 47) | CCAGCATCGTAGGAAGAAAAGCC (SEQ ID NO: 48) |
| *AADAC* | CGGTATTTCTGGAGATAGTGCAG (SEQ ID NO: 49) | TCAAGAGGCTGAAGGGCAGGAT (SEQ ID NO: 50) |
| *NR1I2* | GCTGTCCTACTGCTTGGAAGAC (SEQ ID NO: 51) | CTGCATCAGCACATACTCCTCC (SEQ ID NO: 52) |
| *SULTE1* | CTGCATCAGCACATACTCCTCC (SEQ ID NO: 53) | CCAGGATTTGGATGACCAGCCA (SEQ ID NO: 54) |
| *DUOX2* | CCAGGATTTGGATGACCAGCCA (SEQ ID NO: 55) | GTCCTTGGAGAGGAAGCCATTC (SEQ ID NO: 56) |
| *SLC6A20* | CAGCGAGATGTTCCCGCAAATC (SEQ ID NO: 57) | GCCTCTGTGTAGACGATGAATGC (SEQ ID NO: 58) |
| *SLC43A1* | GATGCTGGAGTACCTTGTGACTG (SEQ ID NO: 59) | CAGGTGAGAAGGCACAACAGCT (SEQ ID NO: 60) |
| *DPP4* | CAAATTGAAGCAGCCAGACA (SEQ ID NO: 61) | GGAGTTGGGAGACCCATGTA (SEQ ID NO: 62) |
| *DEFA5* | CCTTTGCAGGAAATGGACTC (SEQ ID NO: 63) | GGACTCACGGGTAGCACAAC (SEQ ID NO: 64) |
| *ZO-1* | TGTGAGTCCTTCAGCTGTGGAA (SEQ ID NO: 65) | GGAACTCAACACACCATTG (SEQ ID NO: 66) |
| *OCLD* | CATTGCCATCTTTGCCTGTG (SEQ ID NO: 67) | AGCCATAACCATAGCCATAGC (SEQ ID NO: 68) |
| *CLDN1* | CCCAGTCAATGCCAGGTACG (SEQ ID NO: 69) | GGGCCTTGGTGTTGGGTAAG (SEQ ID NO: 70) |
| *CLDN3* | CAGGCTACGACCGCAAGGAC (SEQ ID NO: 71) | GGTGGTGGTGGTGGTGTTGG (SEQ ID NO: 72) |
| *CLDN5* | GCAGCCCCTGTGAAGATTGA (SEQ ID NO: 73) | GTCTCTGGCAAAAAGCGGTG (SEQ ID NO: 74) |
| *ACE2* | TCCATTGGTCTTCTGTCACCCG (SEQ ID NO: 75) | AGACCATCCACCTCCACTTCTC (SEQ ID NO: 76) |
| *PTGES* | GAGGATGCCCTGAGACACGGA (SEQ ID NO: 77) | CCAGAAAGGAGTAGACGAAGCC (SEQ ID NO: 78) |
| *PTGER1* | ATGGTGGTGTCGTGCATCT (SEQ ID NO: 79) | CGCTGCAGGGAGGTAGAG (SEQ ID NO: 80) |
| *PTGER2* | CCACCTCATTCTCCTGGCTA (SEQ ID NO: 81) | AGGTCCCATTTTTCCTTTCG (SEQ ID NO: 82) |
| *PTGER3* | ATCATGTGCGTGCTGTCG (SEQ ID NO: 83) | TGCAGTGCTCAACTGATGTCT (SEQ ID NO: 84) |
| *PTGER4* | CTCCCTGGTGGTGCTCAT (SEQ ID NO: 85) | GGCTGATATAACTGGTTGACGA (SEQ ID NO: 86) |
| *N gene* | GACCCCAAA ATCAGCGAAAT (SEQ ID NO: 87) | TCTGGTTACTGCCAGTTGAATCTG (SEQ ID NO: 88) |
| *E gene* | AGCAGTACGCACACAATCG (SEQ ID NO: 89) | TTCGGAAGAGACAGGTACGTTA (SEQ ID NO: 90) |
| *RdRP* | CTCCTCTAGTGGCGGCTATT (SEQ ID NO: 91) | AGAATAGAGCTCGCACCGTA (SEQ ID NO: 92) |

### Experimental Example 10. Immunofluorescence Test of Cells and Tissues

An immunofluorescence test was performed according to a known method (Kwak et al., Biochemical and biophysical research communications 457, 554-560, 2015). Specifically, the two-dimensional intestinal stem cell population and differentiated intestinal epithelial cells or intestinal tissues were fixed with 4% of paraformaldehyde (PFA) and permeabilized with PBS containing 0.10 of Triton X-100.

The differentiated intestinal epithelial cells or intestinal tissues were cryoprotected with sucrose, and then the membrane of an Insert well was cleaved and vertically placed into an optimal cleavage temperature (OCT) compound (Sakura Finetek, Tokyo, Japan), followed by freezing. Frozen sections were then cut into 10 µm sections using a cryostat microtome at -20°C and permeabilized with PBS containing 0.10 of Triton X-100 for the immunofluorescence test.

After blocking with 4% BSA, the cells were incubated with a primary antibody overnight at 4°C. Thereafter, the mixture was incubated with a secondary antibody at room temperature for 1 hour. The primary antibodies used are shown in Table 2. DAPI was added to visualize nuclei. Slides were observed using an EVOS FL Auto2 (ThermoFisher), an Axiovert 200M microscope (Carl Zeiss, Gottingen, Germany) or a fluorescence microscope (IX51, Olympus, Japan).

**[Table 2]**

| Antibodies | Catalog No. | Company | Dilution |
|---|---|---|---|
| anti-LDHB | PA5-96736 | Thermo Scientific | 1:200 for IF |
| anti-EIF3E | NBP1-84869 | NOVUS | 1:100 for IF |
| anti-SOX9 | sc-166505 | Santa Cruz | 1:100 for IF |
| anti-KI67 | 556003 | BD | 1:100 for IF |
| anti-CD44 | ab6124 | abcam | 1:200 for IF |
| anti-KRT20 | ab76126 | abcam | 1:100 for IF |
| anti-Villin1 | sc-7672 | Santa Cruz | 1:50 for IF |
| anti-Mucin2 | sc-7314 | Santa Cruz | 1:50 for IF |
| anti-Lysozyme | ab76784 | abcam | 1:200 for IF |
| anti-Chromogranin A | MA5-14536 | Thermo Scientific | 1:100 for IF |
| anti-ECAD | AF648 | R&D systems | 1:200 for IF |
| anti-FABP1 | 13368 | Cell signaling Technology | 1:100 for IF |
| anti-Cytokeratin Pure CAM 5.2 | 349205 | BD Biosciences | 25 µg/mL for IF |
| anti-ACE2 | AF933 | R&D systems | 1:100 for IF |

### Experimental Example 11. Single Cell RNA Sequencing

The two-dimensional intestinal stem cell population was washed 2 to 3 times with PBS (Sigma-aldrich), and then 0.25% of TE (Invitrogen) was added thereto, and the cells were detached with a sufficient time of 10 minutes or longer, and then isolated into single cells using 40 µm cell strainer (BD Bioscience). The cells were diluted with PBS comprising 0.04% of BSA, and then the number of cells and viability were measured using CountessIII cell counter (Thermo Scientific. Inc.). To construct a transcript library, a Chromium Next GEM Single Cell 3' reagent kit v3.1 (10X Genomics) was used. Briefly, about 5,000 single-cell transcript libraries were produced by diluting the cells in Chromium Next GEM Chip G, and then about 60,000 base sequences were analyzed per cell using Novaseq 6000 sequencer (Illumina).

### Experimental Example 12. RNA Sequencing and RNA Quantification

For RNA sequencing and quantification, first, RNA samples were prepared with an RNA integrity number (RIN) value of 7.5 or more through an Agilent 2100 Bioanalyzer system (Agilent Biotechnologies, Palo Alto, USA), and a mRNA library was prepared through an Illumina TruSeq kit. Sequencing was performed using Illumina HiSeq2500 machines (Illumina, San Diego, CA, USA). A sequencing quality was determined through a FastQC package, and a trimmed read length of 50 bases or less was excluded. Thereafter, mapping was performed through HISAT2 (v2.0.5), and hg19 was used for human genome information. Genes differentially expressed between samples were analyzed through Cuffquant and Cuffnorm (Cufflinks v2.2.1).

### Experimental Example 13. Bioinformatic Analysis

For analysis of results of single-cell transcript sequencing, initial data were processed using 10X Genomics software CellRanger (version 3.1) and a gene expression matrix was constructed. In addition, the results of analysis of fetal and adult intestinal epithelial tissue single cell transcripts reported in the reference (Elmentaite et al. 2020) were used for transcript comparison analysis. Normalization & feature selection were performed on integrated data using Scanpy package v1.8, and clustering and cell type annotation were performed using the primarily processed data. Thereafter, data integration was performed using spearman's correlation, and a composition ratio for each cell was calculated.

Bioinformatic analysis was performed using IPA analysis software (Ingenuity systems, Redwood City, CA, USA), protein analysis through evolutionary relationships (PANTHER, http://www.pantherdb.org) database, and DAVID bioinformatics resource 6.7 (http://david.abcc.ncifcrf.gov). Functionally grouped gene ontology (GO)/pathways were analyzed using the Cytoscape software platform (Cytoscape software platform, version 3.3.0, http://apps.cytoscape.org/apps/cluego) in conjunction with ClueGO plug-in (Version 2.2.5, http://www.cytoscape.org/what_is_cytoscape.html) .

### Experimental Example 14. Production of Fluorescent Protein-Expressing Cell Line Through Lentivirus Infection

To produce an intestinal stem cell line expressing a fluorescent protein (Green fluorescence protein; eGFP), a lentivirus expressing EF-1α-Gene X-IRES2-EGFP-IRES-Puro was purchased in GeneCopoeia (MD, USA). Approximately 2-4 X 10⁵ two-dimensional intestinal stem cells were centrifuged in a medium comprising a lentivirus and 8 µg/ml of polybrene at 2,500 rpm for 90 minutes, and then an additional medium was added thereto and cultured for 48 hours. For clonal selection, the cells were cultured in a culture medium including 1 µg/ml of puromycin until only colonies expressing the fluorescent protein remained.

To obtain clones derived from single cells, the cells were isolated into single cells by TE treatment, and then the cells were plated at a low density and cultured until a size of the colonies became a certain size or more. Collagenase type IV and Dispase were mixed for 5 minutes to harvest colonies as a whole, and a single colony was isolated by pipetting. The isolated single colonies were transferred to a new plate and cultured to be grown to a sufficient size, and were differentiated into 3-dimensional organoids in a Matrigel dome to confirm differentiation ability of the intestinal stem cell line expressing the fluorescent protein or differentiated into 2.5-dimensional intestinal epithelial cells using air-liquid interface culture.

### Experimental Example 15. Transplantation Experiment of Two-Dimensional Intestinal Stem Cell Population Using Colonoscopy

To confirm the tissue regeneration ability of the two-dimensional intestinal stem cell population, an intestinal epithelial damage model was prepared using hot-EDTA in a male NIG mouse (NOD/SCID deleted IL2Rg gene, 6-12 weeks old; GHBio, Daejeon, Korea). 1.5 X 10⁶ two-dimensional intestinal stem cells per mouse were transplanted using colon endoscopic injectors (Image 1 Hub HD H3-Z; D-Light C; Rigid HOPKINS telescope; Karl Storz, Tuttlingen, Germany; and optimised injector; Vetcom, Gwacheon, Korea) (Matrigel transplantation group, n = 3; intestinal stem cell population transplantation group, n = 5). After transplantation, the cells were blocked with Vetbond Tissue Adhesive (3M, MN, USA) for 6 to 12 hours. Thereafter, the transplanted site was monitored using a colonoscopy on days 0, 3, and 14, and intestinal tissue was isolated from a euthanized mouse to confirm regenerative ability on day 14.

### Experimental Example 16. Tissue Analysis Using Fluorescent Stereomicroscope

To confirm the presence of intestinal stem cells grown in damaged intestinal epithelial tissue, bright field images and fluorescence images of mouse intestine 14 days after transplantation were captured using a stereomicroscope (SZX16, Olympus, Japan).

### Experimental Example 17. Histological (Hematoxylin&Eosin (H&E)) Staining Experiment

For histopathological analysis, intestinal tissues or intestinal epithelial cells were cryoprotected with sucrose, and then the membrane of an insert well was cleaved and vertically placed into an optimal cleavage temperature (OCT) compound (Sakura Finetek, Tokyo, Japan), followed by freezing. Frozen sections were then cut into 10 µm sections using a cryostat microtome at -20°C, adhered to slide glass, and subjected to H&E staining according to the published method. The slides were observed using an optical microscope (BX53F, Olympus, Japan).

### Experimental Example 18. Experiment of Measuring Epithelial Transepithelial electrical resistance (TEER)

To confirm barrier functionality of the differentiated intestinal epithelial cells, the epithelial cells and electrical resistance were measured using an epithelial tissue volt/ohmmeter (EVOM, WPI, FL, USA). All of upper and lower layers of the transwell in which intestinal epithelial cells were cultured were washed with PBS, and then a new culture medium was added thereto, electrodes were immersed in the upper and lower layers one by one, and then a TEER value was measured.

### Experimental Example 19. SARS-CoV-2 Virus Infection Experiment

Differentiated intestinal epithelial cells were infected with 0.01, 0.001 multiplicity of infection (MOI) of SARS-CoV-2 virus produced in vero cells for 1 hour. Thereafter, a medium including the virus was carefully removed, and then a new culture medium was added and further cultured for 72 hours. Thereafter, the cells were harvested for RNA isolation purification to detect virus infected with intestinal epithelial cells.

### Experimental Example 20. Statistical Analysis

All results were expressed as mean ± standard error (s.e.m.) for the mean, and all experiments were repeated at least three times. P values were determined using a two-tailed t-test or single-tailed ANOVA. All analysis of statistical significance was calculated by comparison with the control unless otherwise specified.

### Example 1. Isolation and Culture of Intestinal Stem cell population from Three-Dimensional Intestinal Organoids

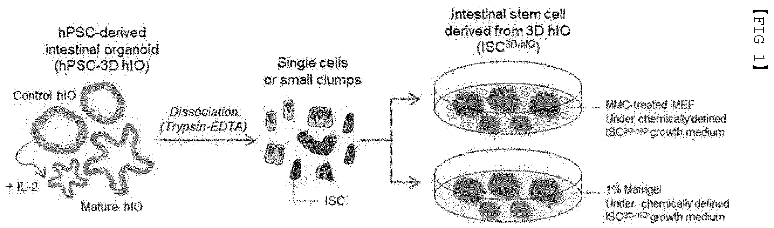

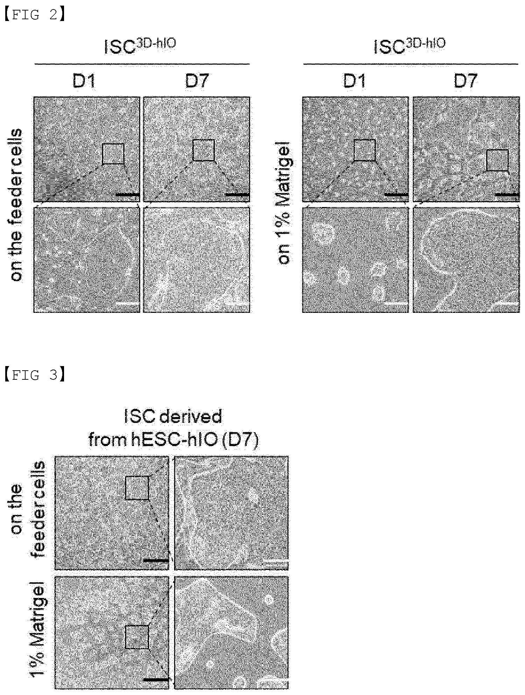

To easily and rapidly large-scale culture high-purity intestinal stem cells, a method for isolating only intestinal stem cells from the three-dimensional intestinal organoids and concentrating the intestinal stem cells was newly constructed (FIG. 1). The newly constructed technology of culturing intestinal stem cells allows the two-dimensional culture of intestinal stem cell population on feeder cells or on plates coated with 1% of Matrigel, and allows isolation and culture of only intestinal stem cells from various types of pluripotent stem cell line-derived three-dimensional intestinal organoids (FIGS. 2 and 3).

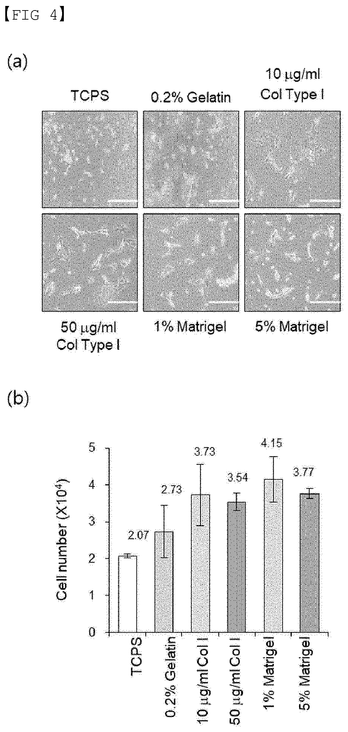

As a result of conducting a coating test using various coating materials in order to maximize an engraftment rate of the intestinal stem cell population, it was confirmed that the engraftment rate was the highest when 1% of Matrigel was coated (FIG. 4). However, it was confirmed that even when gelatin, collagen, or the like is used, the excellent engraftment rate was shown, so that the culture of the intestinal stem cell population may be easily performed without xenobiotic components.

### Example 2. Development of Culture Medium with Optimized Composition for Culturing Two-Dimensional Intestinal Stem Cell Population

To minimize a difference in performance between batches of the two-dimensional intestinal stem cell population, screening was performed to exclude use of chemically undefined factors and to newly discover a culture medium composition comprising factors having a clear composition and capacity.

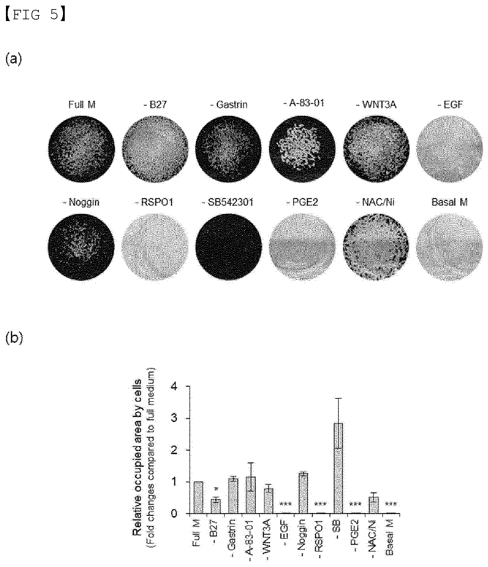

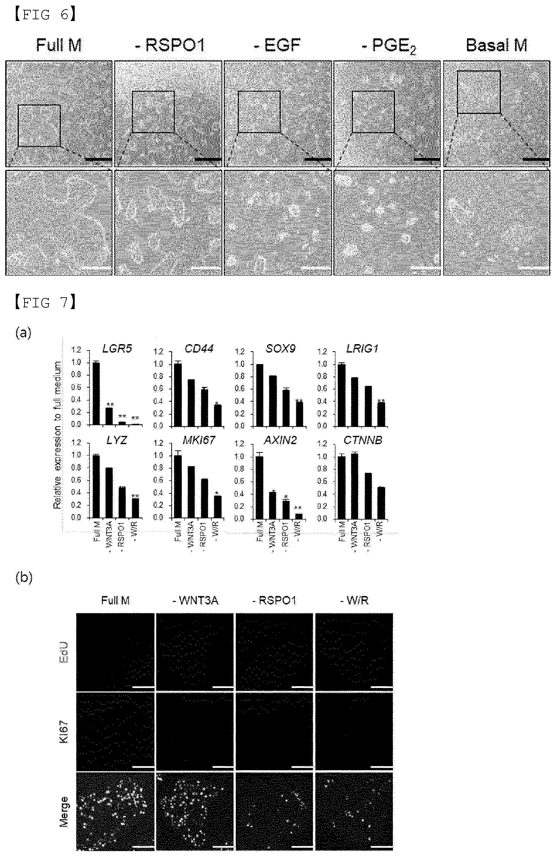

As a result, it was confirmed that the WNT/R-spondin activator, the activator of the prostaglandin signaling pathway, and the receptor tyrosine kinase ligand play an essential role in survival of the intestinal stem cell population. In particular, with regard to these signaling systems, it was confirmed that the combination of R-spondin 1, PGE2, and EGF corresponds to the combination of most suitable factors for the survival of the two-dimensional intestinal stem cell population (FIGS. 5 and 6).

To confirm the role of the above essential factors in detail, a difference in effect according to the presence or absence of each factor was examined in more detail.

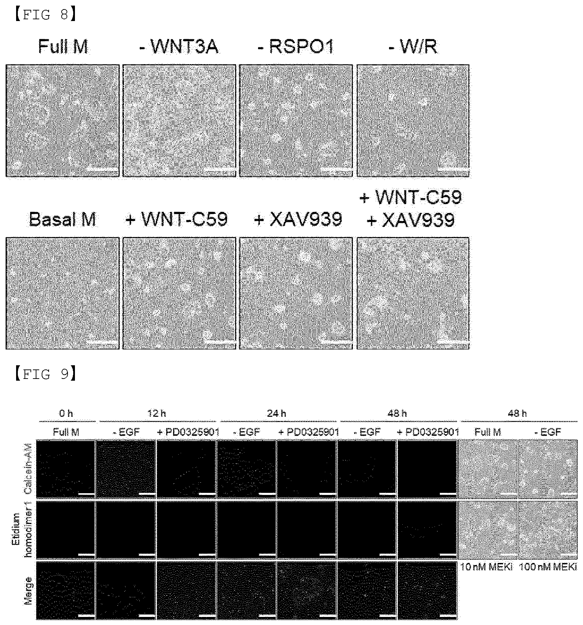

Among the essential factors, R-spondin 1 was found to modulate stemness and proliferation of two-dimensional intestinal stem cells through activation of the WNT signaling pathway (FIGS. 7 and 8).

In addition, EGF was found to prevent death of intestinal stem cell population and modulate proliferation through activation of the EGF-EGFR signaling system (FIG. 9).

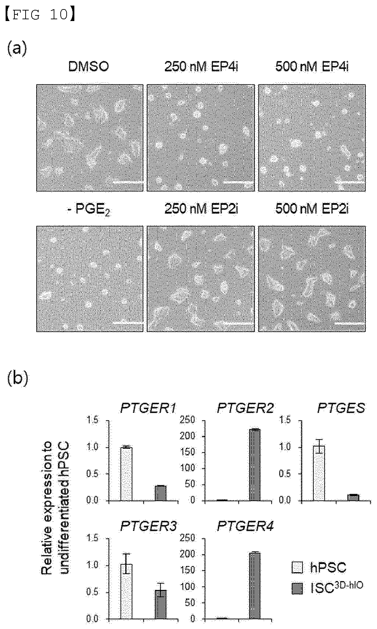

Finally, PGE2 was found to modulate the proliferation of the intestinal stem cell population through activation of the PGE2-EP2/4 signaling pathway (FIG. 10).

### Example 3. Construction of Subculture and Cryostorage Methods of Two-Dimensional Intestinal Stem Cell Population

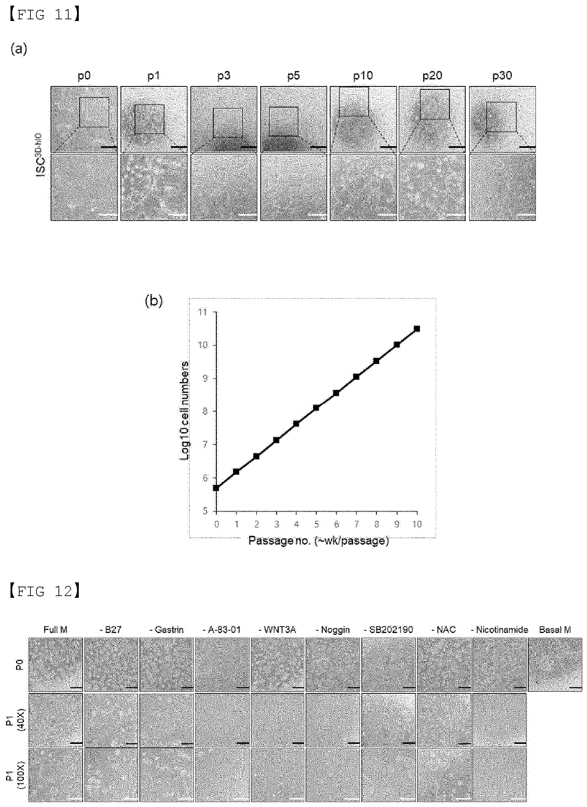

To increase utility of the two-dimensional intestinal stem cell population, stable long-term culture, large-scale culture, cryopreservation, and thawing were tested. First, it can be confirmed that subculture was stably possible more than 30 times in the optimized medium, and mass proliferation was possible without cell loss (FIG. 11).

On the other hand, other factors except essential factors among culture medium composition factors did not affect engraftment and initial proliferation of the two-dimensional intestinal stem cell population. However, it was confirmed that for long-term subculturing, factors such as B27, Noggin, Gastrin, WNT3A, A-83-01, SB202190, N-acetylcysteine, and nicotinamide were required to more efficiently perform long-term culturing (FIG. 12).

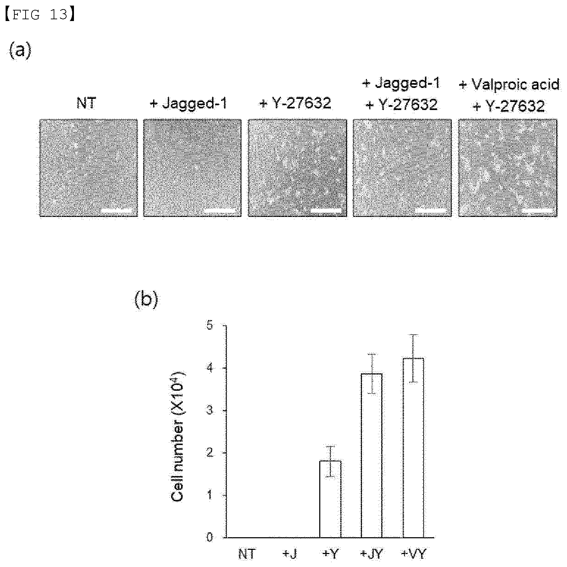

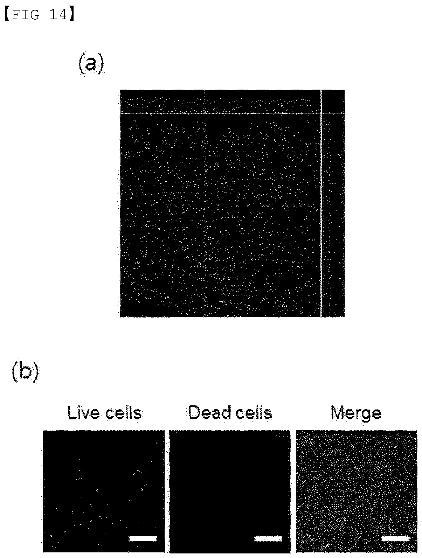

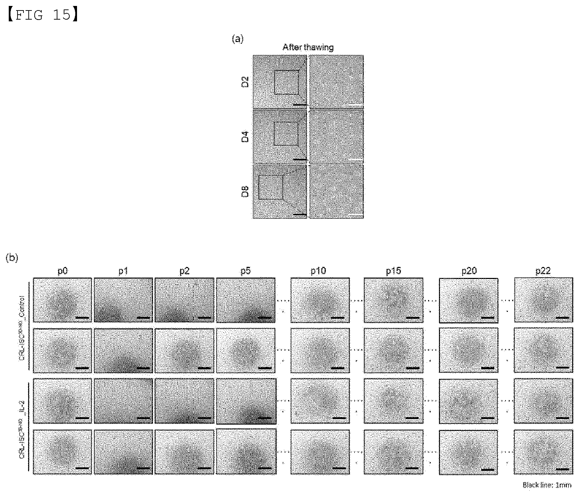

In addition, in order to prevent cell loss during subculture, additional factors important for subculture were discovered. As a result, it can be confirmed that subculture efficiency is increased when a Jagged-1 or valproic acid is added to activate a Notch signaling system, or a Y-27632 is added to inhibit ROCK activity, and it can be confirmed that the subculture efficiency is maximized when two signals are simultaneously controlled (FIG. 13). Under optimized conditions, it was confirmed that the two-dimensional intestinal stem cell population was formed of a monolayer, showed a survival rate of 1000, and was stably cultured (FIG. 14), and it was confirmed that cryopreservation and thawing are possible (FIG. 15(a)), and it was also confirmed that long-term subculture was possible without the difference between batches (FIG. 15(b)).

### Example 4. Method for Analyzing Single Cell Transcript (scRNA-seq) to Analyze Characteristics of Two-Dimensional Intestinal Stem Cell Population

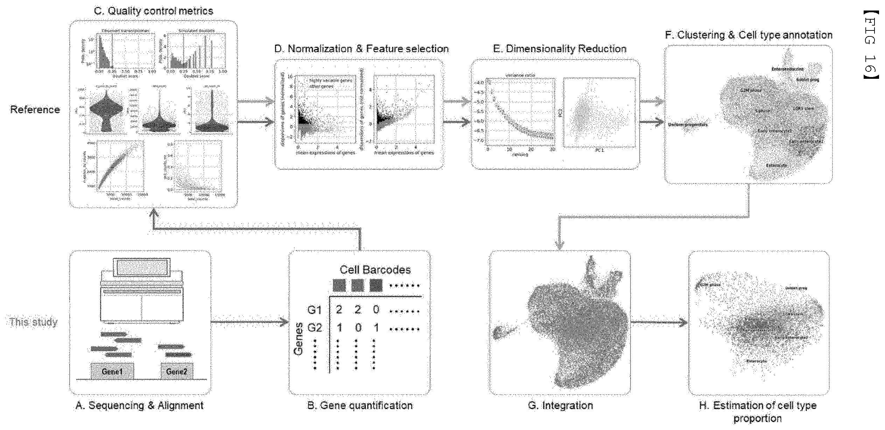

Single-cell transcript sequence analysis was performed to analyze characteristics of the two-dimensional intestinal stem cell population isolated and cultured from the three-dimensional intestinal organoids, and transcript expression pattern analysis and an analysis method for comparison with the results of previous studies reported in the reference document were newly designed (FIG. 16).

### Example 5. Verification of Cell Characteristics and Composition of Two-Dimensional Intestinal Stem Cell Population Using Single Cell Transcript Analysis Results

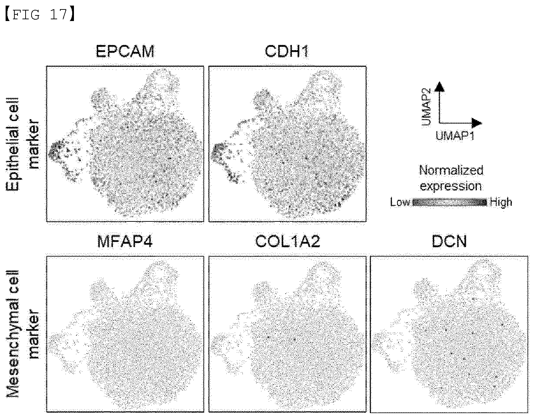

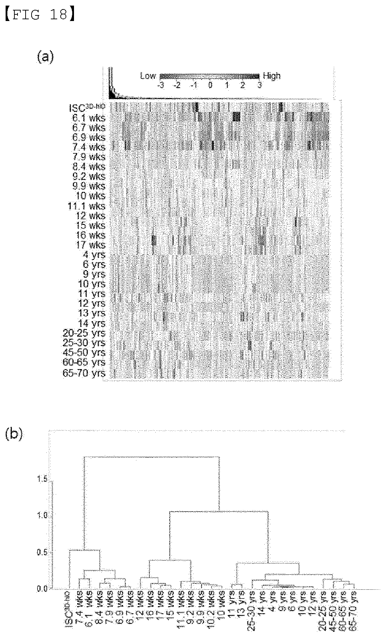

As a result of analyzing characteristics of the two-dimensional intestinal stem cell population based on the single-cell transcript analysis result, it was confirmed that almost all of the two-dimensional intestinal stem cell population exhibited characteristics of intestinal epithelial cells and few cells exhibited characteristics of intestinal mesenchymal stromal cells based on the marker gene expression analysis (FIG. 17). The two-dimensional intestinal stem cell population exhibiting the characteristics exhibited the most similar characteristics to those of human fetal intestinal epithelium when compared to the results of single-cell transcript analysis of human intestinal epithelial tissue at the developmental stages reported in the reference (FIG. 18).

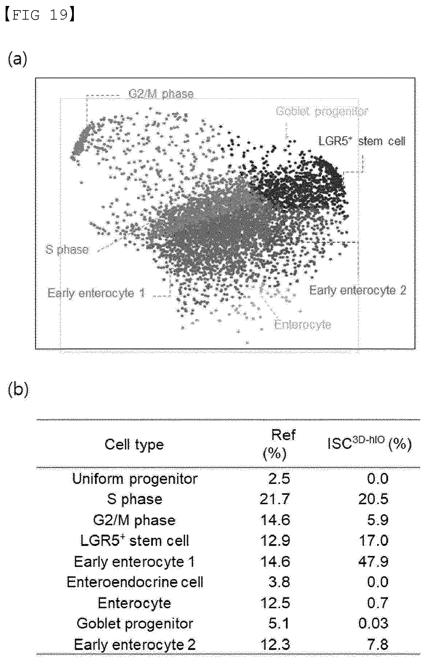

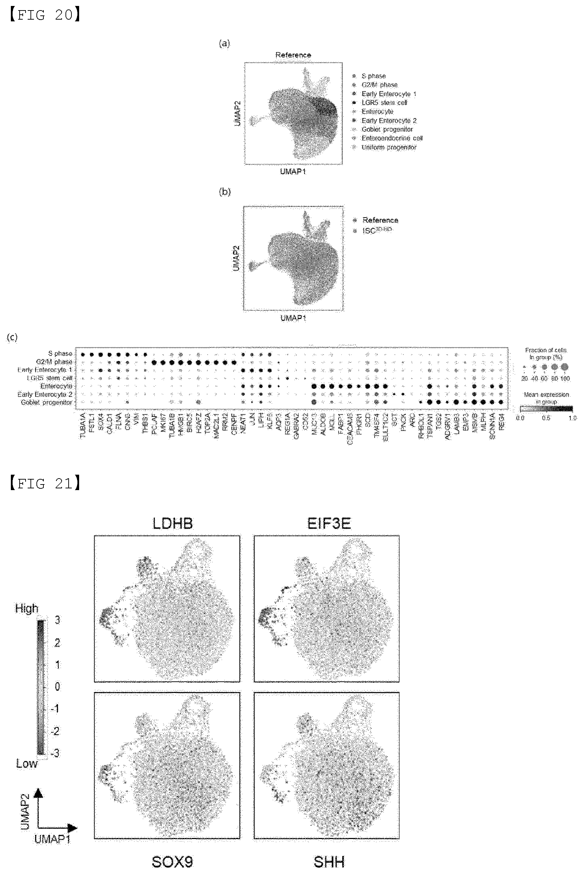

In addition, it was confirmed that the cells constituting the two-dimensional intestinal stem cell population were mainly composed of stem cells and progenitor cells among the cells showing the characteristics of intestinal epithelial cells, and it was confirmed that 900 or more of the total cells were stem cells and progenitor cells (FIG. 19). When analysis was performed by comparing the results of the analysis of the two-dimensional intestinal stem cell population with the results of the analysis of the single-cell transcript of the fetal intestinal epithelial tissue disclosed in the reference, it was confirmed that most of the cells were distributed in a group in which intestinal stem cells and progenitor cells were distributed (FIG. 20).

In addition, as a result of confirming an expression distribution of marker genes (LDHB, EIF3E, SOX9, and SHH) known as markers for intestinal stem cells and progenitor cells, it was confirmed that all marker genes were expressed in almost all cells, so that it was verified that most two-dimensional intestinal stem cell population exhibit characteristics of intestinal stem cells or progenitor cells (FIG. 21).

### Example 6. Verification of Cell Composition of Two-Dimensional Intestinal Stem cell population Using Immunofluorescence Staining

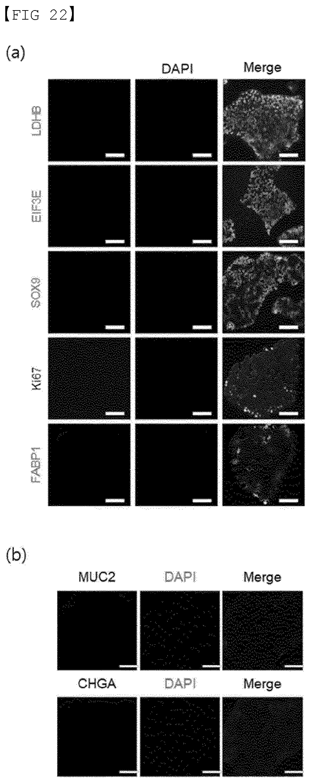

To confirm characteristics of the two-dimensional intestinal stem cell population, as a result of confirming a cell composition using immunofluorescence staining, it was confirmed that marker proteins (LDHB, EIF3E, and SOX9) of the intestinal stem cells and progenitor cells were expressed in most cells, and it was confirmed that some of the cells were actively proliferated (KI67+ cells) (FIG. 22(a)). On the other hand, it was confirmed that the marker protein (FABP1) of absorptive cells in differentiated cells was expressed in some cells, but the marker proteins (MUC2 and CHGA) of secretory cells were not observed (FIGS. 22(a) and (b)).

From the results, it was confirmed that the two-dimensional intestinal stem cell population is mostly composed of a plurality of stem cells and progenitor cells, and very few differentiated cells exist.

### Example 7. Differentiation of Two-Dimensional Intestinal Stem Cell Population into Intestinal Epithelial Cells Using Air-Liquid Interface Culture

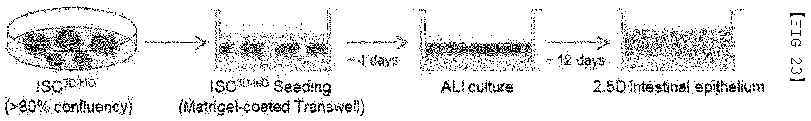

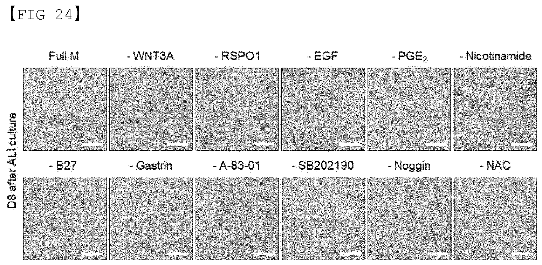

To differentiate the two-dimensional intestinal stem cell population into highly functional intestinal epithelial cells, a differentiation method using air-liquid interface culture was newly constructed (FIG. 23). In this case, medium factor screening was carried out to construct an optimal medium for optimizing differentiation of the two-dimensional intestinal stem cell population into intestinal epithelial cells, and a minimum medium composition could be found by discovering five essential components (FIG. 24).

Specifically, it was confirmed that the activator of the prostaglandin signaling pathway, the receptor tyrosine kinase ligand, the p38 inhibitor, the WNT/R-spondin activator, and the nicotinamide are essential factors for differentiation into intestinal epithelial cells. In particular, treatment with R-spondin 1, EGF, PGE2, SB202190 and nicotinamide was confirmed as an essential factor exhibiting an excellent effect on differentiation into intestinal epithelial cells, which was used as a minimal medium composition.

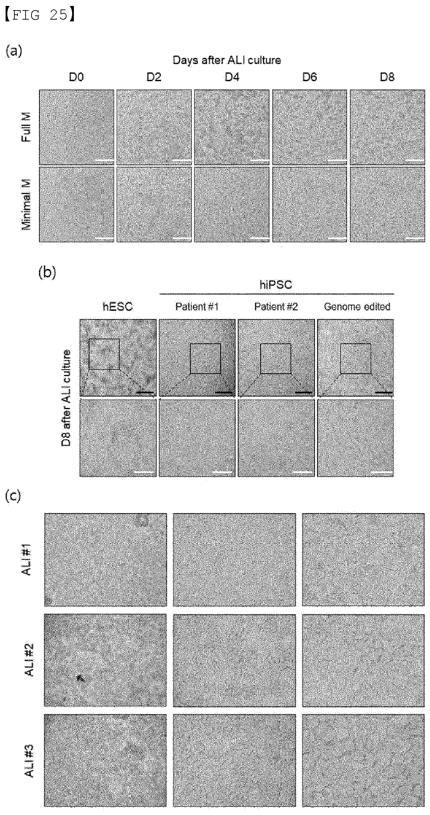

It was confirmed that the differentiation occurred when the two-dimensional intestinal stem cell population was differentiated into intestinal epithelial cells in the confirmed minimum medium, similarly to when the two-dimensional intestinal stem cell population was differentiated in a two-dimensional intestinal stem cell population culture medium. Specifically, it was confirmed that all two-dimensional intestinal stem cell populations derived from various types of pluripotent cell lines were successfully differentiated into intestinal epithelial cells (FIG. 25).

### Example 8. Analysis of Intestinal Epithelial Cell Characteristics Through Marker Gene Expression Analysis

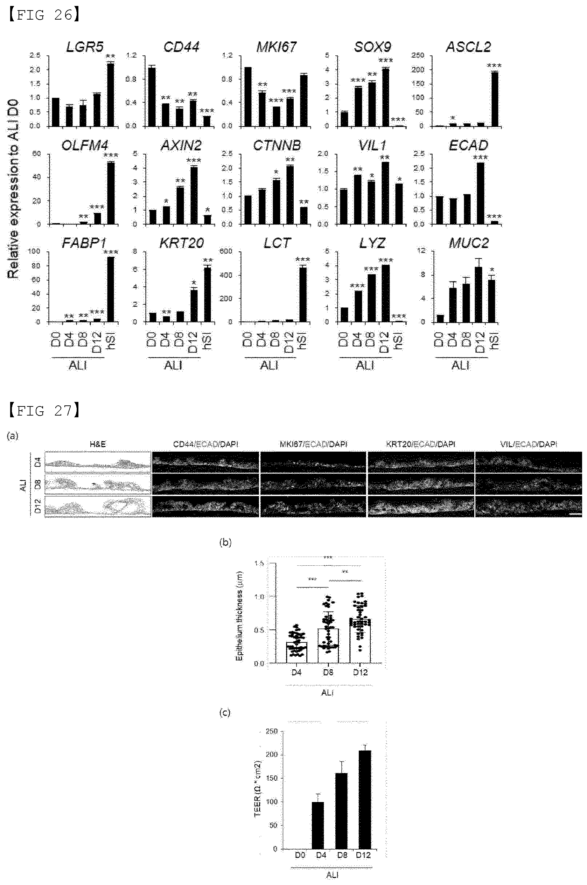

To analyze characteristics of intestinal epithelial cells differentiated by air-liquid interface culture, expression patterns of marker genes specifically expressed in intestinal stem cells and epithelial cells were examined. When the expression of genes in intestinal epithelial cells was examined through qPCR, it was confirmed that the expression of some stem cell marker genes was decreased, and the expression level of most of the differentiated cell marker genes was increased (FIG. 26) .

In addition, as a result of confirming a cross-sectional shape and an expression pattern of the marker protein by collecting the cells on days 4, 8, and 12 after differentiation into intestinal epithelial cells, it was confirmed that a structure similar to crypt-villus was developed in a cross-section of the intestinal epithelial cells over time and a height of the intestinal epithelium increased (FIGS. 27(a) and (b)). Simultaneously, as the intestinal epithelial cells gradually developed over time, it was confirmed that the expression level of the marker protein of differentiated cells gradually increased proportionally (FIG. 27(a)). As described above, it was confirmed through measurement of TEER values that the degree of differentiation of intestinal epithelial cells increased over time and a barrier function of intestinal epithelial cells also continuously increased (FIG. 27 (c)). Therefore, it was confirmed that the intestinal stem cell population was successfully differentiated into intestinal epithelial cells by air-liquid interface culture.

### Example 9. Analysis of Characteristics of Intestinal Stem Cell Population and Intestinal Epithelial Cells Through Transcript Comparison of Various Intestinal Epithelial Cells

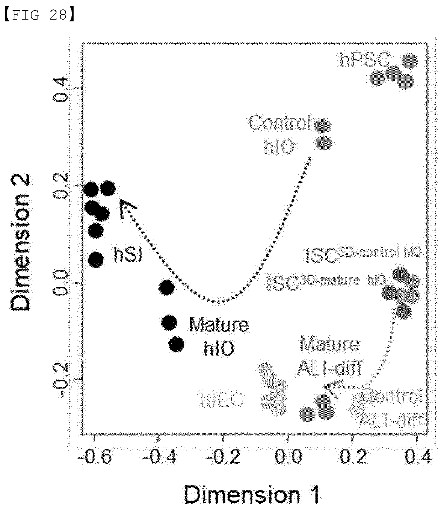

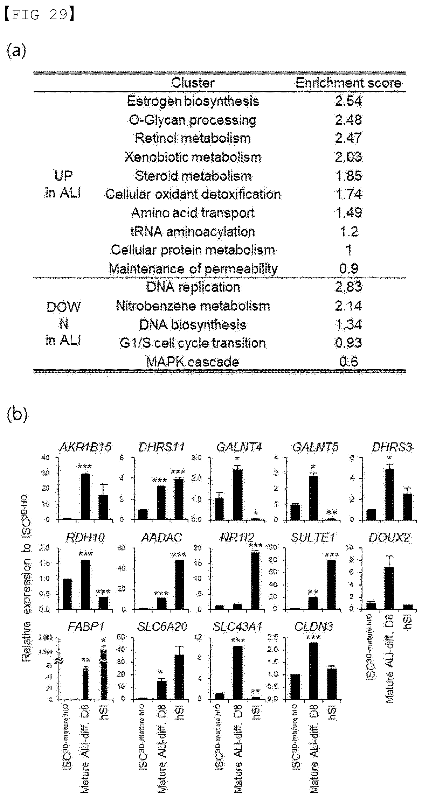

When analysis was performed by comparing expression patterns of pluripotent stem cells, the three-dimensional intestinal organoids derived therefrom, functional intestinal epithelial cells, and transcripts of actual human intestinal tissue, it was confirmed that the two-dimensional intestinal stem cell population was isolated into cells showing different characteristics, apart from other cells (FIG. 28). However, when the two-dimensional intestinal stem cell population was differentiated into intestinal epithelial cells using air-liquid interface culture, it was confirmed that the transcript expression pattern was changed similar to that of other intestinal epithelial cells, and in particular, the cells were grouped most closely to the functional intestinal epithelial cells (FIG. 28). In this case, when the gene group having the largest difference in expression level between the two-dimensional intestinal stem cell population and the intestinal epithelial cells was identified, it was confirmed that the two-dimensional intestinal stem cell population exhibited high expression of genes related to cell proliferation, and the intestinal epithelial cells exhibited high expression of metabolic genes (FIG. 29). Through the results, it was confirmed that the two-dimensional intestinal stem cell population exhibits characteristics of stem cells that activate cell proliferation, and that intestinal epithelial cells may act as differentiated cells having a function of metabolizing various nutrients or substances.

### Example 10. Development of Intestinal Stem Cell Lines Expressing Fluorescent Proteins Using Lentivirus

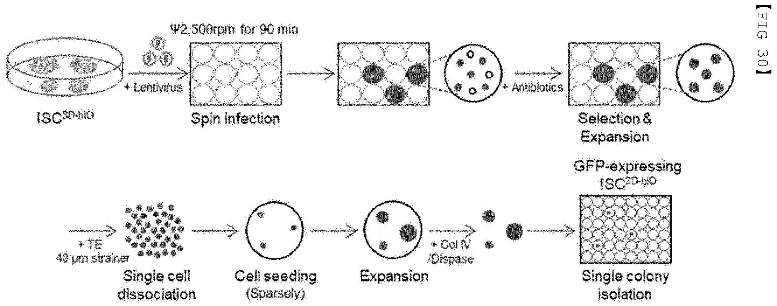

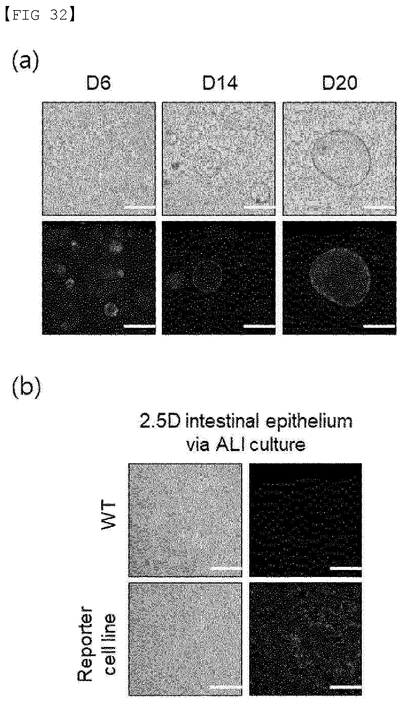

To confirm various utility of the two-dimensional intestinal stem cell population, it was confirmed whether gene-edited cell lines may be produced through introduction of external genes (FIG. 30). First, in order to introduce an external gene, lentivirus including a fluorescent protein was infected into the two-dimensional intestinal stem cell population, and the fluorescent protein was injected into the two-dimensional intestinal stem cell population. Thereafter, in order to select only cells expressing the fluorescent protein, positive selection using antibiotics was performed, and a monoclonal derived from a single cell was also produced using the selected cells (FIG. 31). To verify functionality of the constructed single clone, it was confirmed that when the single clone is differentiated into three-dimensional organoids in a Matrigel dome or into intestinal epithelial cells using air-liquid interface culture, the differentiation is performed in the same manner as normal cells (FIG. 32). From the results, it can be verified that a gene-edited cell line may be produced using the two-dimensional intestinal stem cell population.

### Example 11. Experiment of Verifying Regenerative Ability of Two-Dimensional Intestinal Stem Cell Population Using an Intestinal Epithelial Tissue Damaged Mouse Model

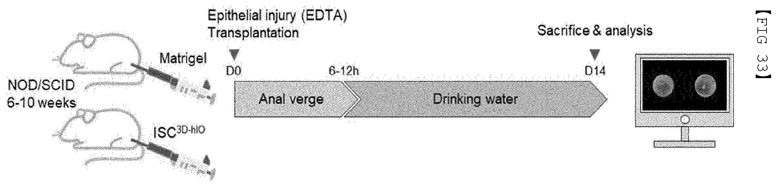

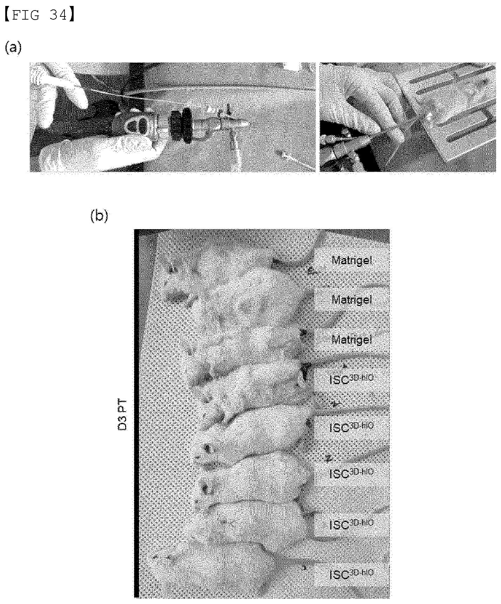

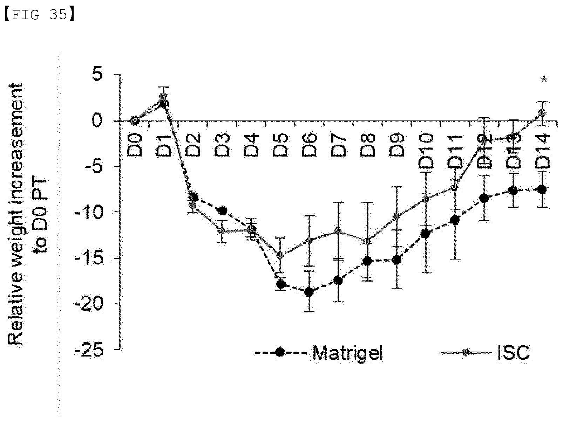

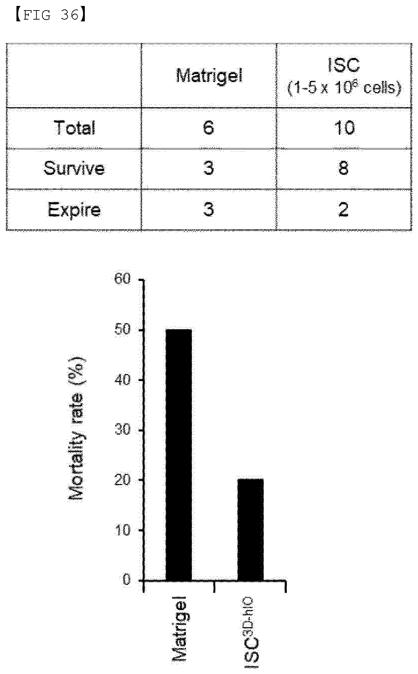

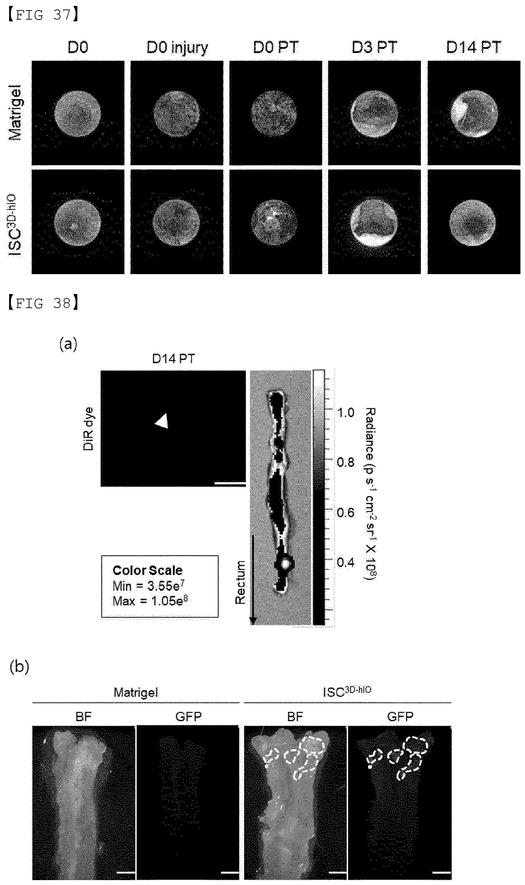

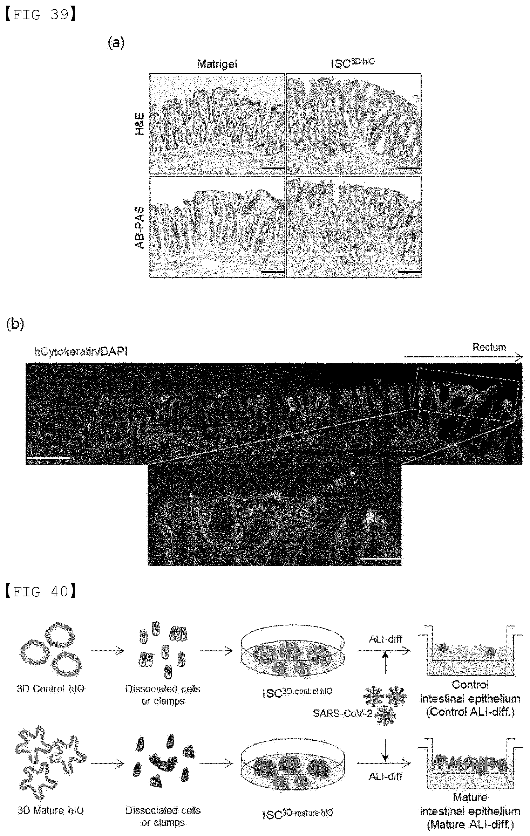

To verify the applicability of the two-dimensional intestinal stem cell population as a cell therapeutic agent, an experiment was conducted by transplanting the population into a mouse model with damaged intestinal epithelial tissue induced by hot-EDTA (FIG. 33). The two-dimensional intestinal stem cell population was transplanted into the lesion using a murine colonoscopy (FIG. 34). It was observed that mice transplanted with the two-dimensional intestinal stem cell population exhibited a faster body weight recovery rate and a higher survival rate compared to a Matrigel-transplanted control group (FIGS. 35 and 36). Colonoscopy observations before and after transplantation confirmed that the lesion recovered more quickly and with a lower immune response when the two-dimensional intestinal stem cell population was transplanted (FIG. 37). This efficacy is attributed to successful engraftment of the two-dimensional intestinal stem cell population in the lesion (FIG. 38), which promotes the regeneration of damaged intestinal tissue (FIG. 39). Therefore, it was confirmed that the two-dimensional intestinal stem cell population effectively regenerates the lesion when transplanted into damaged intestinal epithelial tissue, demonstrating its potential as a biomaterial for use as a cell therapeutic agent in intestinal epithelial tissue regeneration treatment.

### Example 12. SARS-CoV-2 Infectious Disease Modeling Using 2.5-Dimensional Intestinal Epithelial Cell Model

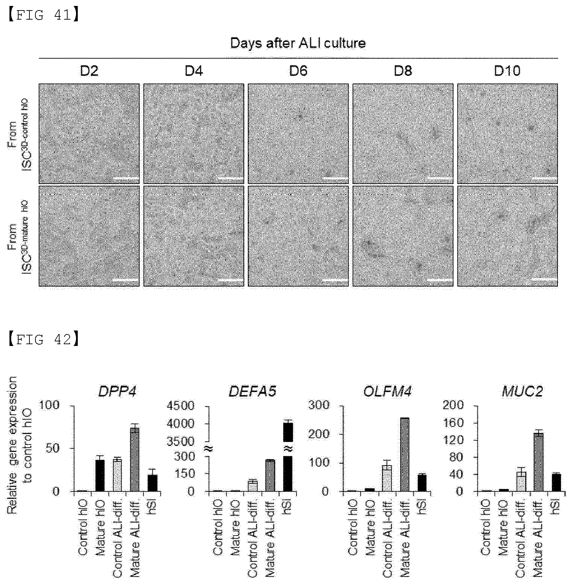

To verify the utilization of 2.5-dimensional intestinal epithelial cells derived from the two-dimensional intestinal stem cell population, infectious disease modeling using SARS-CoV-2 virus was performed (FIG. 40). To confirm whether there is a difference in sensitivity to SARS-CoV-2 virus infection according to maturation of intestinal epithelial cells, the two-dimensional intestinal stem cell population was isolated from immature/mature three-dimensional intestinal organoids, and immature/mature intestinal epithelial cells were produced using air-liquid interface culture. As a result of monitoring cell morphology for 10 days after air-liquid interface culture, it was confirmed that there was no difference between immature and mature intestinal epithelial cells (FIG. 41), but there was a difference in expression of a maturation-related marker gene (FIG. 42).

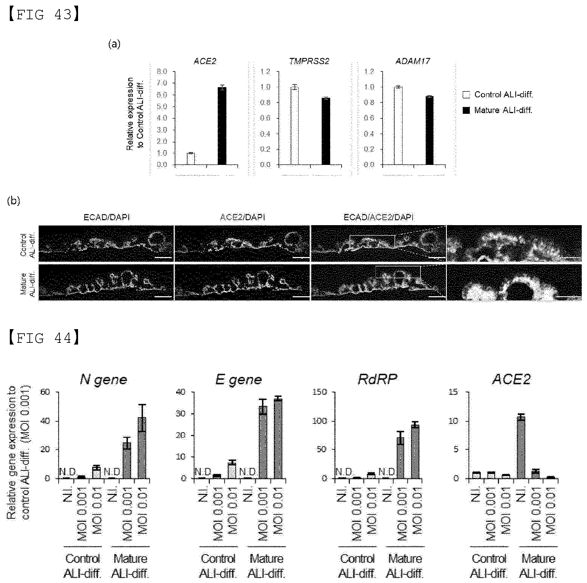

In particular, it was confirmed that an expression level of ACE2 among receptors important for SARS-CoV-2 virus infection was higher in the mature intestinal epithelial cells (FIG. 43), and thus it was confirmed that SARS-CoV-2 infection was more sensitively infected in the mature intestinal epithelial cells (FIG. 44).

Based on these results, it was confirmed that the 2.5-dimensional intestinal epithelial cells derived from the two-dimensional intestinal stem cell population may be used as a cell model for modeling various diseases including infectious diseases.

## Claims

1. A method for culturing an intestinal stem cell population, the method comprising:
(a) isolating a pluripotent stem cell-derived three-dimensional intestinal organoid into a single cell or a small cell clump; and
(b) two-dimensionally culturing the single cell or the small cell clump in a culture medium comprising a WNT/R-spondin activator, an activator of a prostaglandin signaling pathway, and a receptor tyrosine kinase ligand.

2. The method of claim 1, wherein the WNT/R-spondin activator is any one or more selected from the group consisting of R-spondin 1, R-spondin 2, R-spondin 3, R-spondin 4, and R-spondin mimetic substances.

3. The method of claim 1, wherein the activator of the prostaglandin signaling pathway is any one or more selected from the group consisting of arachidonic acid (AA), prostaglandin E2 (PGE2), prostaglandin G2 (PGG2), prostaglandin F2 (PGF2), prostaglandin H2 (PGH2) and prostaglandin D2 (PGD2).

4. The method of claim 1, wherein the receptor tyrosine kinase ligand is any one selected from the group consisting of an epidermal growth factor (EGF), a transforming growth factor-alpha (TGF-alpha), a basic fibroblast growth factor (bFGF), a brain-derived neurotrophic factor (BDNF), a hepatocyte growth factor (HGF), and a keratinocyte growth factor (KGF).

5. The method of claim 1, wherein the culture medium in (b) further comprises any one or more selected from the group consisting of B27, N-acetyl-L-cysteine (NAC), nicotinamide, Gastrin, a TGF-beta inhibitor, a Wnt signaling pathway activator, a BMP inhibitor, and a p38 inhibitor.

6. The method of claim 5, wherein the TGF-beta inhibitor is any one selected from the group consisting of A-83-01, SB-431542, SB-505124, SB-525334, SD-208, LY-36494, and SJN-251.

7. The method of claim 5, wherein the Wnt signaling pathway activator is any one selected from the group consisting of Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16.

8. The method of claim 5, wherein the BMP inhibitor is Noggin, Dorsomorphin, DMH1, or LDN-193189.

9. The method of claim 5, wherein the p38 inhibitor is any one selected from the group consisting of SB202190, SB203580, SB239063, SB706504, BIR796, JX401, EO1428, RWJ67657, SCIO469, VX745, TAK715, ML3403, DBM1285, and PH797804.

10. The method of claim 1, wherein the culture medium of (b) further comprises a ROCK inhibitor, a Notch activator, or both of the ROCK inhibitor and the Notch activator at an initial stage of culture.

11. The method of claim 1, wherein the culture medium in (b) is selected from the group consisting of Dulbecco's modified eagle medium (DMEM), Ham's F12, DMEM/F12, DMEM/F12, and Advanced DMEM/F12.

12. The method of claim 1, wherein the intestinal stem cell population exhibits an enhanced expression level for any one or more markers selected from the group consisting of LGR5, CD44, SOX9, LRIG1, LYZ, AXIN2, CTNNB, and MKI67.

13. The method of claim 1, wherein the intestinal stem cell population exhibits any one or more markers selected from the group consisting of LDHB, EIF3E, SOX9, and SHH.

14. The method of claim 1, wherein the intestinal stem cell population comprises 800 or more of cells comprising a S phase cell, an LGR5+ stem cell, and an early enterocyte, with respect to total cells of the population.

15. The method of claim 1, wherein the pluripotent stem cell-derived three-dimensional intestinal organoid is produced through:
(a) culturing pluripotent stem cells in a medium comprising any one or more selected from the group consisting of Nodal, Activin A, Activin B, BMP4, Wnt3a, CHIR99021, and bFGF to differentiate the stem cells to definitive endoderm;
(b) culturing the definitive endoderm in a medium comprising: any one or more GSK3 inhibitors selected from the group consisting of BIO(6-bromoindirubin-3'-oxime), SB216763 (3-(2,4-dichlorophenyl)-4-(1-methyl-1H-indol-3-yl)-1H-pyrrole-2,5-dione), GSK-3β inhibitor VII(α,4-dibromoacetophenone), L803-mts(Myr-N-GKEAPPQSpP-NH₂), and CHIR99021; and a fibroblast growth factor (FGF), to differentiate the definitive endoderm into three-dimensional hindgut spheroids; and
(c) culturing the three-dimensional hindgut spheroids in a medium comprising: a BMP inhibitor; a WNT/R-spondin activator; a receptor tyrosine kinase ligand; and any one or more factors selected from the group consisting of IL-2, IL-22, IL-6, IL-1β, IL-11, EGF, OSM, NRG-1, IL-10, and colivelin, to produce a three-dimensional intestinal organoid.

16. The method of claim 15, wherein in (c), the medium further comprises one or more selected from the group consisting of a ROCK inhibitor, B27, N-acetyl-L-cysteine (NAC), N2, nicotinamide, Gastrin, IGF-1, heregulin-1β, bFGF, A-83-01, and SB202190.

17. The method of claim 1, wherein in the isolating of the pluripotent stem cell-derived three-dimensional intestinal organoid into the single cell or the small cell clump, the intestinal organoid is treated with EDTA, trypsin, or both of the EDTA and the trypsin and is dissociated into a single cell or a small cell clump.

18. An intestinal stem cell population produced by the culturing method of any one of claims 1 to 17.

19. A method for producing an intestinal epithelial cell, the method comprising:
(a) isolating a pluripotent stem cell-derived three-dimensional intestinal organoid into a single cell or a small cell clump;
(b) two-dimensionally culturing the single cell or the small cell clump in a culture medium comprising a WNT/R-spondin activator, an activator of a prostaglandin signaling pathway, and a receptor tyrosine kinase ligand to produce an intestinal stem cell population; and
(c) culturing the intestinal stem cell population in a differentiation medium comprising an activator of a prostaglandin signaling pathway, a receptor tyrosine kinase ligand, a p38 inhibitor, an WNT/R-spondin activator, and nicotinamide by air-liquid interface culture.

20. The method of claim 19, wherein the air-liquid interface culture is culturing the intestinal stem cell under a transwell coated with an extracellular matrix.

21. The method of claim 19, wherein the WNT/R-spondin activator is any one or more selected from the group consisting of R-spondin 1, R-spondin 2, R-spondin 3, R-spondin 4, and R-spondin mimetic substances.

22. The method of claim 19, wherein the activator of the prostaglandin signaling pathway is any one or more selected from the group consisting of arachidonic acid (AA), prostaglandin E2 (PGE2), prostaglandin G2 (PGG2), prostaglandin F2 (PGF2), prostaglandin H2 (PGH2), and prostaglandin D2 (PGD2).

23. The method of claim 19, wherein the receptor tyrosine kinase ligand is any one selected from the group consisting of an epidermal growth factor (EGF), a transforming growth factor-alpha (TGF-alpha), a basic fibroblast growth factor (bFGF), a brain-derived neurotrophic factor (BDNF), a hepatocyte growth factor (HGF), and a keratinocyte growth factor (KGF).

24. The method of claim 19, wherein the p38 inhibitor is any one selected from the group consisting of SB202190, SB203580, SB239063, SB706504, BIR796, JX401, EO1428, RWJ67657, SCIO469, VX745, TAK715, ML3403, DBM1285, and PH797804.

25. The method of claim 19, wherein the intestinal epithelial cell exhibits an enhanced expression level for any one or more markers selected from the group consisting of VIL1, ECAD, FABP1, KRT20, LCT, AXIN2, LYZ, and MUC2.

26. The method of claim 19, wherein the intestinal epithelial cell exhibits an enhanced expression level for any one or more markers selected from the group consisting of AKR1B15, DHRS11, GALNT4, GALNT5, DHRS3, RDH10, AADAC, NR1I2, SULTE1, DOUX2, FABP1, SLC6A20, SLC43A1, and CLDN3.

27. The method of claim 19, wherein the intestinal epithelial cell comprises a small intestinal cell, a mucus secretory cell, a hormone secretory cell, and a paneth cell.

28. An intestinal epithelial cell produced by the producing method of any one of claims 19 to 27.

29. An intestinal epithelial cell model comprising the intestinal epithelial cell of claim 28.

30. The intestinal epithelial cell model of claim 29, wherein the intestinal epithelial cell model has a crypt-villus structure.

31. A drug screening method comprising:
(a) infecting the intestinal epithelial cell model of claim 29 with bacteria or viruses;
(b) treating the infected intestinal epithelial cell model with a drug; and
(c) confirming a reaction according to drug treatment.

32. A drug evaluation method comprising:
(a) performing drug treatment on the intestinal epithelial cell model of claim 29; and
(b) evaluating absorbance or bioavailability of a drug in the intestinal epithelial cell model in (a).

33. A method for providing an intestinal epithelial cell model for disease modeling, the method comprising infecting the intestinal epithelial cell model according to claim 29 with bacteria or viruses.

34. A tissue therapeutic agent comprising the intestinal stem cell population of claim 18.

35. A pharmaceutical composition for assisting intestinal transplantation comprising the intestinal stem cell population of claim 18.
